# EUROPEAN PATENT APPLICATION

(11) **EP 4 190 341 A1**
(43) Date of publication of application: **07.06.2023**
(21) Application number: 21212602.3
(22) Date of filing: 06.12.2021
(51) Int. Cl.: A61K 36/35, A61P 31/12

(54) **ELDERBERRY EXTRACT FOR USE IN A METHOD OF PREVENTING OR TREATING SARS-COV-2 INFECTION**

(71) Applicant: Iprona AG/SpA, 39011 Lana (IT)
(72) Inventor: SCHUBERT, Ulrich, 07646 Trockenborn-Wolfersdorf (DE); PLATTNER, Stephan, 39010 Nals (IT); STEINKASSERER, Alexander, 91352 Hallerndorf (DE); SETZ, Christian, 90562 Kalchenreuth (DE)
(74) Representative: Engelhard, Markus

(57) **Abstract**

The present invention relates to an elderberry extract for use in a method of preventing or treating a SARS-CoV-2 infection, wherein said elderberry extract is an ultrafiltrated elderberry extract. The present invention further relates to a composition, preferably pharmaceutical composition, comprising an elderberry extract. Furthermore, the present invention relates to a method of preparing an ultrafiltrated elderberry extract.

## Description

### FIELD OF THE INVENTION

The present invention relates to an elderberry extract for use in a method of preventing or treating a SARS-CoV-2 infection, wherein said elderberry extract is an ultrafiltrated elderberry extract. The present invention further relates to a composition, preferably pharmaceutical composition, comprising an elderberry extract. Furthermore, the present invention relates to a method of preparing an ultrafiltrated elderberry extract.

### BACKGROUND OF THE INVENTION

Severe acute respiratory syndrome coronavirus 2 (SARS□CoV□2) is the virus that causes COVID-19 (coronavirus disease 2019), the respiratory illness responsible for the COVID-19 pandemic. SARS-CoV2 has caused a pandemic resulting in serious problems to the global economy and placing a heavy burden on national health systems on a global scale. SARS-CoV2 infections often times involve intensive care in hospitals. Despite increasing vaccination rates, there are still high numbers of infections, and new SARS-CoV2 variants arise. Particularly in view of newly developing variants, effective treatments and means of prevention are necessary to tackle the COVID-19 pandemic. Thus, there is a need for means for preventing and/or treating SARS-CoV-2 infections. Furthermore, there is a need for immune system boosters which help to improve a body's immune response.

Elderberries comprise a high content of anthocyanins which have been considered to have beneficial health effects for the immune system, particularly for the treatment of infections such as flu and cold. The concentrated juice of elderberry suppressed viral replication in the bronchoalveolar lavage fluids of mice infected with human influenza a virus [1]. Tiralongo [2] suggests that a membrane filtered elderberry extract reduces cold duration and severity in air travelers. Due to a toxic effect of raw elderberries, elderberry should not be consumed in unprocessed form. Components from elderberries may be extracted using ethanol or similar organic solvents. Ethanolic elderberry extract (30, 60, and 90 µg/ml) enhances cell proliferation, and promotes alkaline phosphatase (ALP) and bone Gla protein (BGP) activities, and type I collagen (Col-I) synthesis in primary cultured osteoblasts via BMP-2/Smad/p38/JNK/Runx2 signaling pathway [3]. However, beneficial components of elderberries may be lost during processing of the fruit raw material. Particularly, polysaccharides, vitamins and micronutrients are poorly extracted and concentrated using conventional anthocyanin extraction and purification methods, and may be lost during processing of elderberry fruits. Thus, there is the need to provide elderberry products, particularly an extract, that comprises a matrix of desirable components, such as anthocyanins, polysaccharides, micronutrients and vitamins of elderberries. Furthermore, there is the need to provide extracts of elderberries with standardized amounts of anthocyanins. There is also the need for methods to provide elderberry extracts, particularly a gentle process which allows to preserve the desired ingredients of elderberries.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to an elderberry extract for use in a method of preventing or treating a SARS-CoV-2 infection, wherein said elderberry extract is an ultrafiltrated elderberry extract, preferably an ultrafiltrated black elderberry extract, optionally of *Sambucus nigra L* and/or *Sambucus canadensis L.*

In one embodiment, said extract
comprises one or more anthocyanins, preferably selected from the group consisting of cyanidin 3-glucoside, cyanidin 3-sambucoside, cyanidin 3-sambubioside 5-glucoside, and combinations thereof, and/or
comprises one or more polyphenols, preferably selected from rutin, quercetin, catechin, and epicatechin.

In one embodiment, said extract comprises one or more polyphenols, wherein said polyphenols are selected from anthocyanins, preferably anthocyanins selected from the group consisting of cyanidin 3-glucoside, cyanidin 3-sambucoside, and cyanidin 3-sambubioside 5-glucoside; flavonoids, preferably flavonoids selected from rutin and quercetin; and catechins, preferably catechins selected from catechin and epicatechin.

In one embodiment, said extract has an anthocyanin concentration of at least 1 wt%, preferably at least 3 wt%; wherein, optionally, said extract has an anthocyanin concentration in the range of from 2.5 wt% to 20 wt%, preferably from 3 wt% to 18 wt%, e.g. 3.2 wt%, 4 wt%, 7 wt%, 10 wt%, 14 wt%, or 15%.

In one embodiment, said extract has a high molecular weight fraction concentration of at least 1 wt%, preferably at least 3 wt%, more preferably in the range of from 5 wt% to 10 wt%,
wherein, preferably, said high molecular weight fraction has a molecular weight in a range of from 5 kDa to 2 MDa,
wherein, optionally, said high molecular weight fraction comprises protein(s), dietary fibers, and/or polysaccharide(s).

In one embodiment, said extract has a dietary fiber concentration of at least 1 wt%, preferably of at least 2 wt%,
wherein, optionally, said dietary fiber is selected from pectins and polysaccharides, e.g. acidic polysaccharides, and combinations thereof.

In one embodiment, said extract is in a liquid form or in a solid form,
wherein, optionally, said extract in a solid form has an anthocyanin concentration of at least 1.5 wt%, preferably of at least 10 wt%.

In one embodiment, said extract is an extract ultrafiltrated with an ultrafiltration membrane having a molecular weight cut-off of at least 400 Da, preferably of at least 500 Da, more preferably of at least 1000 Da.

In one embodiment, in said use, said extract is administered to a patient in need thereof in the form of a solid extract at a dose in the range of from 30 mg to 10.000 mg per day, preferably at least 250 mg per day, more preferably at least 900 mg per day; or in the form of a liquid extract at a dose in the range of from 100 µL to 1 L per day, preferably 1 mL to 100 mL per day, more preferably at least 5 mL per day.

In one embodiment, in said use, said extract is administered to a patient in need thereof in the form of a formulation selected from a capsule; a tablet, e.g. an effervescent tablet or a chewable tablet; a syrup; a sachet; gummies; a lozenge; a shot; a gel; a confection; a spray, e.g. an oral spray; a powder; granules; a solution; a suspension; a dispersion; an emulsion; a bar; a drink; a liquid concentrate; an aerosol; or drops;
wherein, optionally, said formulation further comprises, in addition to said extract, an excipient, e.g. a pharmaceutically acceptable excipient, a sweetener, a sugar, a starch, a preservative, a flavoring agent, a nutritional oil such as a vegetable oil or fish oil, water, a milk such as a cow's milk, a soya milk, or an almond milk, a black elderberry juice or other fruit juice, a vegetable juice, and/or a colorant.

In one embodiment, said extract stimulates increased expression of IL-6, TNF-a, and/or IL-23, preferably IL-6, TNF-a, and IL-23, in immune cells, and/or
said extract inhibits a viral replication of said SARS-CoV-2.

In one embodiment, in said use, said extract is administered to a patient in need thereof at one or several stages selected from
- a healthy stage;
- an asymptomatic disease stage;
- a mild or moderate disease stage;
- a severe disease stage; and
- a critical disease stage.

In one embodiment, said SARS-CoV-2 infection is an infection with a SARS-CoV-2 wildtype, preferably severe acute respiratory syndrome coronavirus 2 isolate Wuhan-hu-1, and/or a SARS-CoV-2 variant, preferably alpha variant or beta variant.

In one embodiment, said SARS-CoV-2 variant is selected from alpha variant, beta variant, gamma variant, and delta variant, and/or is selected from
B.1.1.7 variant, B.1.351 variant, P.1 variant, B.1.617 variant, B.1.617.1 variant, B.1.617.2 variant, B.1.617.3 variant, B.1.427 variant, B.1.429 variant, B.1.525 variant, B.1.526 variant, P.2 variant, and any combination thereof;
wherein, preferably, said SARS-CoV-2 variant is selected from alpha variant and beta variant.

In a further aspect, the present invention relates to a composition, preferably pharmaceutical composition, comprising an elderberry extract, wherein said elderberry extract is an ultrafiltrated elderberry extract, optionally an elderberry extract as defined above;
optionally, said extract has an anthocyanin concentration of at least 1 wt%, preferably of at least 3 wt%, e.g. in the range of from 2.5 wt% to 20 wt%, preferably from 3 wt% to 18 wt%;
optionally, said extract has a high molecular weight fraction concentration of at least 1 wt%, preferably of at least 3 wt%, wherein, preferably, said high molecular weight fraction has a molecular weight in a range of from 5 kDa to 2 MDa;
optionally, said extract comprises dietary fiber(s) in a concentration of at least 1 wt%, preferably of at least 2 wt%;
optionally, said composition further comprises an excipient, e.g. a pharmaceutically acceptable excipient, a sweetener, a sugar, a starch, a preservative, a flavoring agent, a nutritional oil such as a vegetable oil or fish oil, water, a milk such as a cow's milk, a soya milk, or an almond milk, an elderberry juice or other fruit juice, a vegetable juice, and/or a colorant, preferably a pharmaceutically acceptable excipient.

In a further aspect, the present invention relates to a method of preparing an ultrafiltrated elderberry extract, comprising the steps:
i) providing one or more elderberries; wherein, preferably, said one or more elderberries comprise black elderberries; wherein, more preferably, said one or more elderberries comprise *Sambucus nigra L* and/or *Sambucus canadensis L*;
ii) performing an ultrafiltration using an ultrafiltration membrane having a molecular weight cut-off of at least 400 Da, preferably of at least 500 Da, more preferably of at least 1000 Da;
iii) obtaining an ultrafiltrated elderberry extract.

In one embodiment, said method further comprises a step of performing a water extraction of said one or more elderberries of step i) prior to performing said ultrafiltration of step ii). In one embodiment, said water extraction comprises mixing said one or more elderberries with water and mechanically disrupting said one or more elderberries, e.g. by juicing, mashing, grinding, shredding, chopping, pressing, blending, and/or pulping said one or more elderberries.

In one embodiment, said elderberry extract, said ultrafiltration, and said ultrafiltrated elderberry extract are as defined above.

In a further aspect, the present invention relates to a method of preventing or treating a SARS-CoV-2 infection, comprising administering to a patient in need thereof an effective amount of an elderberry extract and/or of a composition comprising an elderberry extract.

In this aspect, said SARS-CoV-2 infection, said elderberry extract, and said composition are as defined above.

In a further aspect, the present invention relates to the use of an elderberry extract in the manufacture of a composition for a method of preventing or treating a SARS-CoV-2 infection.

In this aspect, said SARS-CoV-2 infection, said elderberry extract, and said composition are as defined above.

### DETAILED DESCRIPTION

It is an aim of the invention to provide an extract for use in the treatment of SARS-CoV2 infections. An extract for use of the present invention is prepared in an ultrafiltration-based enrichment process which increases the effectiveness of the extract by increasing the concentration of all the desirable nutrients and preserving the original food matrix. The extract is rich in a vast array of phytonutrients present in the fresh fruits. The extract is highly advantageous in that it provides synergistic actions of the fruit components, such as anthocyanins and a high molecular weight fraction which are useful in treating SARS-CoV2 infections.

In one embodiment, the term "elderberry extract", as used herein, relates to an extract of one or more elderberry fruits, preferably an ultrafiltrated extract of elderberry fruits. In one embodiment, the term "elderberry" relates to elderberry fruit(s). Elderberries comprise about 80 % water, 18 % carbohydrates, and less than 1 % each of protein and fat. Elderberries, preferably black elderberries, are rich in anthocyanins. An elderberry extract for use of the present is prepared by means of ultrafiltration, and is enriched with anthocyanins and/or a high molecular weight fraction comprising dietary fibers. Such enrichment of an extract with anthocyanins and/or a high molecular weight fraction comprising dietary fibers is based on an enrichment of the respective components present in the elderberry raw material using ultrafiltration. Such anthocyanins, high molecular weight fraction, and dietary fibers are not added to said extract, but consist of the anthocyanins, high molecular weight fraction, and dietary fibers present in the elderberry raw material used to prepare the extract. An ultrafiltrated elderberry extract comprises anthocyanins, a high molecular weight fraction, and dietary fibers of elderberry raw material. Such anthocyanins, high molecular weight fraction, and dietary fibers of elderberry raw material are retained within the extract when preparing the ultrafiltrated elderberry extract using ultrafiltration. In one embodiment, when referring to a component such as anthocyanin being "enriched", it means that the respective component was already present in the one or more elderberries used to prepare the ultrafiltrated elderberry extract and that such components are enriched by concentrating the extract using ultrafiltration. In one embodiment, an elderberry extract comprises about 20 wt% polyphenols, about 7 wt% dietary fibers, as well as sugar and peptides. In one embodiment, said elderberry extract is in a liquid form, i.e. is a liquid elderberry extract, and/or in a solid form, i.e. is a solid elderberry extract. In one embodiment, a liquid elderberry extract has an anthocyanin concentration in the range of from 1 wt% to 10 wt%, preferably of from 1 wt% to 5 wt%, more preferably of at least 2.5 wt%. In one embodiment, a solid elderberry extract has an anthocyanin concentration in the range of from 1 wt% to 25 wt%, preferably of from 2.5 wt% to 20 wt%, more preferably of from 5 wt% to 15 wt%. In one embodiment, a liquid extract has an anthocyanin concentration of from 3 wt% to 4 wt%. In one embodiment, a solid extract has an anthocyanin concentration of from 4 wt% to 15 wt%. In one embodiment, a liquid extract is obtained performing an ultrafiltration and has an anthocyanin concentration of 3.2 wt%. In one embodiment, a solid extract is obtained by drying, such as spray-drying, of a liquid extract obtained by ultrafiltration. In one embodiment, a solid extract is a spray-dried extract. In one embodiment, a solid extract, e.g. spray-dried extract, has an anthocyanin concentration of 4 wt%, 7 wt%, 10 wt%, 14 wt%, or 15 wt%. In one embodiment, as spray dried extract is spray dried on a food additive, for example a polysaccharide such as maltodextrin. In many of the embodiments, an elderberry extract is prepared from black elderberry (*Sambucus nigra*), e.g. *Sambucus nigra L.* In one embodiment, an elderberry extract for use of the present invention has immunoregulatory potential. In one embodiment, an elderberry extract of the present invention comprises molecules having a molecular weight of from 400 Da to 2.000.000 Da, preferably 500 Da to 2.000.000 Da, e.g. 500 Da to 70.000 Da. In one embodiment, the terms "solid extract", "solid elderberry extract", and "elderberry extract in a solid form" are used interchangeably. In one embodiment, the terms "liquid extract", "liquid elderberry extract", and "elderberry extract in a liquid form" are used interchangeably.

The term "ethanol extract", as used herein, refers to an elderberry extract prepared using an extraction with an organic solvent, preferably an extraction using ethanol or methanol, more preferably using an ethanol extraction. In one embodiment, the elderberry extract, particularly the ultrafiltrated elderberry extract, of the invention differs from an ethanol extract in at least the composition, e.g. the ethanol extract has a higher anthocyanin concentration than the ultrafiltrated elderberry extract, such as the ethanol extract having an anthocyanin concentration of more than 25 wt%. Typically, ethanol extracts are obtained using chromatography columns and/or resins that specifically enrich and purify anthocyanins. Typically, when preparing an ethanol extract, organic solvents such as ethanol are used to obtain the anthocyanins from the cells and subsequently from the chromatography columns. Thus, ethanol extracts have high concentrations of anthocyanins. However, the concentrations of other components of the elderberry fruit, such as polysaccharides, vitamins, and minerals, are reduced in an ethanol extract compared to an ultrafiltrated extract. Such reduced levels of components, such as polysaccharides, vitamins, and minerals, in an ethanol extract compared to an ultrafiltrated extract are, for example, due to the method of preparing the ethanol extract involving a column and solvents, such as ethanol. The ultrafiltrated extract of the invention is highly advantageous in that the method of production thereof involves water as solvent instead of ethanol a solvent. Thereby, the preservation of comprised components is enhanced. Preparing ethanol extracts typically comprises using solvents to dissolve the active ingredients and anthocyanins from the fruit. The anthocyanins then bind to columns, are washed, and then dissolved from the column with ethanol. The ethanol is then evaporated and recovered. In contrast thereto, the ultrafiltrated extract of the invention is produced without alcoholic solvents such as ethanol. A juice is produced from the elderberry fruit raw material, and the anthocyanins are subsequently enriched by means of ultrafiltration to obtain an extract of the invention.

In one embodiment, the method of preventing or treating a SARS-CoV-2 infection comprises administering said extract to a patient in need thereof in the form of a solid extract at a dose in the range of from 30 mg to 10.000 mg per day, preferably at least 250 mg per day, more preferably at least 900 mg per day; or in the form of a liquid extract at a dose in the range of from 100 µL to 1 L per day, preferably 1 mL to 100 mL per day, more preferably at least 5 mL per day. In one embodiment, the method of preventing or treating a SARS-CoV-2 infection comprises administering said extract to a patient in need thereof in the form of a formulation selected from a capsule; a tablet, e.g. an effervescent tablet or a chewable tablet; a syrup; a sachet; gummies; a lozenge; a shot; a gel; a confection; a spray, e.g. an oral spray; a powder; granules; a solution; a suspension; a dispersion; an emulsion; a bar; a drink; a liquid concentrate; an aerosol; or drops. In one embodiment, said method comprises administering said extract to a patient in need thereof at one or several stages selected from
- a healthy stage;
- an asymptomatic disease stage;
- a mild or moderate disease stage;
- a severe disease stage; and
- a critical disease stage.

In one embodiment, said SARS-CoV-2 infection is an infection with a SARS-CoV-2 wildtype, preferably severe acute respiratory syndrome coronavirus 2 isolate Wuhan-hu-1 or SARS-CoV-2PR-1, and/or a SARS-CoV-2 variant, preferably alpha variant or beta variant. In one embodiment, a SARS-CoV-2 wildtype is severe acute respiratory syndrome coronavirus 2 isolate Wuhan-hu-1 or SARS-CoV-2PR-1. In one embodiment, severe acute respiratory syndrome coronavirus 2 isolate Wuhan-hu-1 is as described in NCBI with accession number NC_045512.2. In one embodiment, severe acute respiratory syndrome coronavirus 2 isolate Wuhan-hu-1 has a sequence as defined in NCBI Reference Sequence NC_045512.2. In one embodiment, SARS-CoV-2PR-1 is as defined in [5].

In one embodiment, the term "ultrafiltrated elderberry extract", as used herein, relates to an elderberry extract prepared using ultrafiltration. Ultrafiltration relates to membrane filtration in which forces like pressure or concentration gradients lead to a separation through a semipermeable membrane. Suspended solids and solutes of high molecular weight are retained in the retentate, while water and low molecular weight solutes pass through the membrane in the permeate (filtrate). Ultrafiltration membranes are defined by the molecular weight cut-off (MWCO) of the membrane used. In one embodiment, the term "ultrafiltrated" relates to an extract having been ultrafiltrated with a ultrafiltration membrane having a molecular weight cut-off of at least 400 Da, preferably of at least 500 Da, more preferably of at least 1000 Da. In one embodiment, an ultrafiltrated elderberry extract is a retentate of an ultrafiltration. In one embodiment, an ultrafiltrated elderberry extract has a distinct composition resulting from the ultrafiltration, e.g. from the molecular weight cut-off. In one embodiment, elderberry fruit components having a molecular weight that exceeds the molecular weight cut-off of the ultrafiltration membrane, e.g. at least 400 Da, preferably at least 500 Da, more preferably at least 1000 Da, are retained in the membrane; for example, anthocyanins, complexes of anthocyanins, and/or high molecular weight fractions are retained in the membrane. In one embodiment, a purely mechanical separation process without application of chemicals or heat is used to prepare an extract, enabling the separation of substances according to their different molecular sizes using a semi-permeable membrane. In one embodiment, the ultrafiltrated elderberry extract is an ultrafiltrated black elderberry extract, preferably of *Sambucus nigra* and/or *Sambucus canadensis.* In one embodiment, the terms "*Sambucus nigra*" and "*Sambucus nigra L*" are used interchangeably. In one embodiment, the terms "*Sambucus canadensis*" and "*Sambucus canadensis L*" are used interchangeably. In one embodiment, the elderberry extract is a black elderberry extract. In one embodiment, the ultrafiltrated elderberry extract is an ultrafiltrated black elderberry extract. In one embodiment, the term "black elderberry", as used herein, relates to an elderberry of the genus Sambucus. Black Elderberries are high in flavonoids such as anthocyanins, which give the berries their bluish-purple color, as well as anthocyanidins. In one embodiment, black elderberries are elderberries of *Sambucus nigra* and/or *Sambucus canadensis.* In one embodiment, *Sambucus nigra* is *Sambucus nigra L.* In one embodiment, *Sambucus canadensis* is *Sambucus canadensis L.*

In one embodiment, the term "anthocyanin", as used herein, relates to any anthocyanin known to person skilled in the art, particularly water-soluble vacuolar pigments that may appear red, purple, or blue. Food plants rich in anthocyanins include the elderberry, aronia, blackcurrent, blueberry, raspberry, black rice, and black soybean. Anthocyanins belong to the flavonoids and are synthesized involving the phenylpropanoid pathway. Anthocyanins are found in the cell vacuole, mostly in fruits and flowers, but also occur in leaves, stems, and roots. In these parts, they are found predominantly in outer cell layers such as the epidermis and peripheral mesophyll cells. Most frequently occurring in nature are the glycosides of cyanidin, delphinidin, malvidin, pelargonidin, peonidin, and petunidin. In one embodiment, the term anthocyanin relates to a glycoside of cyanidin, delphinidin, malvidin, pelargonidin, peonidin, or petunidin, preferably cyanidin. In one embodiment, anthocyanin may be present in the form of a complex and/or aggregate, in which several anthocyanins are aggregated, optionally involving binding of magnesium. The term "cyanidin", as used herein, refers to a particular type of anthocyanin. It is a pigment found in many red berries including grapes, blackberry, blueberry, cherry, cranberry, elderberry, and raspberry. In one embodiment, a cyanidin is selected from cyanidin 3-glucoside, cyanidin 3-sambucoside, cyanidin 3-sambubioside 5-glucoside, and combinations thereof. In one embodiment, the elderberry extract for use of the invention is enriched with anthocyanins, e.g. cyanidin 3-glucoside, cyanidin 3-sambucoside, cyanidin 3-sambubioside 5-glucoside, and combinations thereof. In one embodiment, the elderberry extract for use of the invention comprises anthocyanins, e.g. cyanidin 3-glucoside, cyanidin 3-sambucoside, cyanidin 3-sambubioside 5-glucoside, and combinations thereof.

In one embodiment, said ultrafiltrated extract has an anthocyanin concentration of at least 1 wt%, preferably at least 3 wt%. In one embodiment, said anthocyanin is selected from the group consisting of cyanidin 3-glucoside, cyanidin 3-sambucoside, cyanidin 3-sambubioside 5-glucoside, and combinations thereof. In one embodiment, said extract has an anthocyanin concentration in the range of from 2.5 wt% to 20 wt%, preferably from 3 wt% to 18 wt%, e.g. 3.2 wt%, 4 wt%, 7 wt%, 10 wt%, 14 wt%, or 15%. In one embodiment, components such as the anthocyanins and/or flavonoids comprised in the extract of the invention have an antiviral effect against SARS-CoV-2. In one embodiment, cyanidin 3-sambucoside has an antiviral effect against SARS-CoV-2, e.g. by blocking the neuraminidase enzyme in the viral replication process. In one embodiment, flavonoids have an antiviral effect against SARS-CoV-2, e.g. by blocking receptors on a virus surface. In one embodiment, an anthocyanin concentration is determined using any suitable method known to the person skilled in the art, e.g. HPLC.

In one embodiment, the term "enriched", as used herein, refers to an increased presence of a component in a unit of a processed composition, such as a fruit extract e.g. ultrafiltrated elderberry extract, compared to the initial composition, such as fruit raw material. In one embodiment, the concentration of enriched components, such as anthocyanins and a high molecular weight fraction, is higher in an ultrafiltrated elderberry extract than in fruit raw material. In one embodiment, the amount of anthocyanin is enriched in an elderberry extract compared to the elderberry raw material, e.g. the amount of anthocyanin is enriched in 1 kg of elderberry extract compared to 1 kg of elderberries. In one embodiment, anthocyanin is enriched in an elderberry extract at a ratio of at least 2 to 1, preferably of at least 16 to 1, more preferably of at least 72 to 1, compared to the fruit raw material. In one embodiment, said enrichment refers to 2 kg fruit material to 1 kg extract, 16 kg fruit material to 1 kilo extract, 72 kg fruit material to 1 kg extract, respectively. In one embodiment, a high molecular weight fraction is enriched in an elderberry extract for use of the present invention compared to the raw fruit material. In one embodiment, anthocyanin and/or a high molecular weight fraction is/are enriched in an elderberry extract for use of the present invention compared to the raw fruit material, e.g. at a ratio of 2:1, 16:1, or 72:1. The term "ratio", as used herein, indicates a relation between a processed product, particularly an elderberry extract, and the unprocessed raw material, particularly elderberry fruits. In one embodiment, a ratio indicates the amount of raw material used to prepare a certain amount of processed product. In one embodiment, when referring to a ratio of 2:1, 16:1, and 72:1, said ratios indicate that 2 kg raw fruit material, 16 kg raw fruit material, and 72 kg raw fruit material, respectively, were used to prepare 1 kg of elderberry extract. In one embodiment, when referring to enrichment of anthocyanin at a ratio of 2:1, 16:1, and 72:1, said ratios refer to the same amount of anthocyanin present in 2 kg fruit raw material, 16 kg fruit raw material, and 72 kg fruit raw material, respectively, being present in 1 kg processed product, i.e. in 1 kg of elderberry extract.

The terms "raw material", "fruit material", and "fruit raw material", as used herein, refers to fruits, particularly elderberries, preferably elderberries which have not been subjected to extraction. In one embodiment, fruit raw material refers to unprocessed fruits. In one embodiment, fruit raw material is used to prepare an extract of the present invention involving ultrafiltration. In one embodiment, the one or more elderberries, provided in step i) of a method of preparing ultrafiltrated elderberry extract, is fruit raw material. In one embodiment, fruit raw material refers to fresh fruits and/or frozen fruits. In one embodiment, the term "fresh fruit" may relate to a fruit such as an unprocessed fruit up to five days after harvest, preferably two days after harvest, more preferably up to 12 hours after harvest, and/or may relate to a fruit which has been frozen within 48 hours, preferably within 12 hours, more preferably within 6 hours, more preferably within 3 hours after harvest. In one embodiment, fruit raw material relates to elderberries.

In one embodiment, the term "high molecular weight fraction", as used herein, relates to a fraction of elderberries that is obtained during ultrafiltration of said elderberries. In one embodiment, said high molecular weight fraction has a molecular weight in a range of from 5 kDa to 2 MDa. In one embodiment, the composition of the high molecular weight fraction is determined by the pore size of the membrane used for ultrafiltration. In one embodiment, said ultrafiltration membrane has a molecular weight cut-off of at least 400 Da, preferably of at least 500 Da, more preferably of at least 1000 Da. In one embodiment, said high molecular weight fraction comprises dietary fibers. In one embodiment, any component comprised in a high molecular weight fraction has a molecular weight in a range of from 5 kDa to 2 MDa. In one embodiment, a high molecular weight fraction comprises components such as polysaccharides, oligosaccharides, agglomerated molecules, high molecular weight proteins. In one embodiment, the extract for use of the invention comprises a high molecular weight fraction having a molecular weight of at least 5 kDa, preferably in a range of from 5 kDa to 2 MDa. In one embodiment, all components of the high molecular weight fraction have a molecular weight of at least 5 kDa, preferably in a range of from 5 kDa to 2 MDa. In one embodiment, the extract of the invention has a high molecular weight fraction concentration of at least 1 wt%, preferably at least 3 wt%, more preferably in the range of from 5 wt% to 10 wt%. In one embodiment, said high molecular weight fraction comprises or consists of components that have a molecular weight that exceeds the molecular weight cut-off of at least 400 Da, preferably of at least 500 Da, more preferably of at least 1000 Da. In one embodiment, said high molecular weight fraction comprises aggregates of compounds that are smaller than the molecular weight cut-off of at least 400 Da, preferably of at least 500 Da, wherein said aggregates have a molecular weight that exceeds the molecular weight cut-off of at least 400 Da, preferably of at least 500 Da, more preferably of at least 1000 Da. In one embodiment, said high molecular weight fraction comprises protein(s), dietary fibers, and/or polysaccharide(s). In one embodiment, said high molecular weight fraction comprises or consists of protein(s), dietary fibers, and/or polysaccharide(s). In one embodiment, the high molecular weight fraction of the extract is highly advantageous in that it comprises polysaccharides that have an immune stimulating effect. An advantage of the extract for use of the invention is that it comprises a high molecular weight fraction comprising polysaccharides having an inhibitory effect on viral replication of SARS-CoV2, e.g. SARS-CoV2 wildtype, alpha variant or beta variant.

In one embodiment, the term "dietary fiber", as used herein, refers to any dietary fiber known to the person skilled in the art, particularly a portion of fruits that cannot be completely broken down by digestive enzymes in a body. Dietary fibers may comprise soluble fibers and insoluble fibers. Soluble fibers are water-soluble and are fermented in the colon into gases and physiologically active byproducts such as short-chain fatty acids produced in the colon by gut bacteria. Soluble fibers may delay gastric emptying which can result in an extended feeling of fullness. Insoluble fibers are inert to digestive enzymes in the upper gastrointestinal tract and may absorb water as they move through the digestive system. Insoluble fibers may ease defecation. In one embodiment, dietary fibers are selected from polysaccharides, cellulose, starch, dextrins, inulin, lignin, chitins, pectins, oligosaccharides, and combinations thereof. Consuming dietary fibers has several advantages, namely, firstly, it increases food volume without having a high caloric content, thereby providing satiety which may reduce appetite, secondly, it attracts water and forms a viscous gel during digestion, thereby delaying absorption of glucose which lowers the variance of the blood sugar levels, thirdly, it lowers cholesterol levels which may reduce the risk of cardiovascular disease, fourthly, it alleviates constipation. Furthermore, the ultrafiltrated elderberry extract comprising dietary fibers promotes an immunostimulatory effect. In one embodiment, dietary fibers comprise any pectins, acidic polysaccharides, and other polysaccharides, and combinations thereof. In one embodiment, said extract has a dietary fiber concentration of at least 1 wt%, preferably of at least 2 wt%. In one embodiment, an extract in a solid form has a dietary fiber concentration in the range of from 2 wt% to 20 wt%, preferably 5 wt% to 10 wt%. In one embodiment, an extract in a liquid form has a dietary fiber concentration in the range of from 1 wt% to 10 wt%, preferably 1 wt% to 5 wt%. In one embodiment, said dietary fiber(s) comprise(s) or consist(s) of pectins and/or polysaccharides, e.g. acidic polysaccharides.

The term "molecular weight cut-off', as used herein, relates to a threshold which determines whether a component is retained in a membrane during ultrafiltration, or whether a component is not retained in a membrane. The molecular weight cut-off is it determined by the characteristics of the membrane used for filtration, such as the pore size and/or the material of the membrane. In one embodiment, the molecular weight cut-off is in a range of from 5 kDa to 2 MDa. In one embodiment, an ultrafiltrated extract is a retentate of an ultrafiltration with a membrane having a molecular weight cut-off of at least 400 Da, preferably of at least 500 Da, more preferably of at least 1000 Da, e.g. in the range of from 5 kDa to 2 MDa. The term "patient", as used herein, relates to a human or animal. Said patient may suffer from a disease, such as a SARS-CoV-2 infection, and an extract of the present invention may be used to treat said disease, or said patient may be healthy and said extract may be used to prevent a disease, such as a SARS-CoV-2 infection, and/or to improve the general health condition. The term "administered", as used herein, refers to application of an elderberry extract to a patient. Said application can be performed topically or systemically, such as an oral, nasal, mucosal, intrabronchial, intrapulmonar, intradermal, subcutaneous, intravenous, intramuscular, intravascular, intrathecal, intraocular, intraarticular, and/or intranodal administration. In a preferred embodiment, said elderberry extract is administered orally. Said administration of an elderberry extract can be performed using various routes and dosage forms. In one embodiment, the extract for use of the invention is formulated as a capsule; a tablet, e.g. an effervescent tablet or a chewable tablet; a syrup; a sachet; gummies; a lozenge; a shot; a gel; a confection; a spray, e.g. an oral spray; a powder; granules; a solution; a suspension; a dispersion; an emulsion; a bar; a drink; a liquid concentrate; an aerosol; or drops. In one embodiment, the extract for use of the invention is a solid, semisolid, and/or liquid extract. In one embodiment, an extract is provided as a dietary supplement, e.g. as a component of a food item and/or drink item. In one embodiment, the daily dose for administering an extract for use of the invention to a patient in need thereof is at least 500 mg of anthocyanins, more preferably at least 1000 mg. The term "effective amount", as used herein, refers to an amount of an elderberry extract and/or a composition comprising an elderberry extract which is in the range between the dose sufficient to evoke a therapeutic effect and the maximum tolerated dose. In one embodiment, an effective amount of an elderberry extract to be administered to a patient is in the range of from 30 mg to 10.000 mg, preferably at least 250 mg, more preferably at least 900 mg per day, of a solid extract, and/or is in the range of from 100 µL to 1 L, preferably 1 mL to 100 mL, more preferably at least 5 mL, of a liquid extract. In one embodiment, the composition of the invention is formulated as a capsule; a tablet, e.g. an effervescent tablet or a chewable tablet; a syrup; a sachet; gummies; a lozenge; a shot; a gel; a confection; a spray, e.g. an oral spray; a powder; granules; a solution; a suspension; a dispersion; an emulsion; a bar; a drink; a liquid concentrate; an aerosol; or drops. In one embodiment, the composition of the invention is for use in a method of preventing or treating a SARS-CoV-2 infection.

In one embodiment, the term "liquid form", as used herein, relates to a formulation of said extract, wherein the extract is liquid. For example, a liquid extract has a viscosity of ≤ 10 mPa·s, preferably ≤ 8 mPa s, as measured using a Mettler RM 180 Rheomat at room temperature at 500 RPM with a spindle diameter of 1.4 cm and a container diameter of 3.3cm. The viscosity of a liquid extract can be increased by admixing a thickening agent. In one embodiment, the term "solid form", as used herein, relates to a formulation of said extract, wherein the extract is solid. In one embodiment, an extract in a solid form has an anthocyanin concentration of at least 1.5 wt%, preferably of at least 10 wt%. In one embodiment, an extract in the solid form is a powder. In one embodiment, an extract in a solid form, e.g. powder, is a dried liquid extract. For example, an extract in a liquid form, i.e. liquid extract, is dried via spray drying or lyophilization, preferably spray drying, to obtain an extract in a solid form, i.e. a solid extract. In one embodiment, the components of a solid extract and a liquid extract are similar and/or identical except of the water content. In one embodiment, the drying factor of a solid extract compared to a liquid extract is factor 4, i.e. 4 units of liquid extract, e.g. 4 kg of liquid extract, are dried to obtain 1 unit of solid extract, e.g. 1 kg of solid extract, such as extract in the form of a powder. For example, a liquid extract may have an anthocyanin concentration of 3.2 wt%. For example, a solid extract, e.g. powder extract, may have an anthocyanin concentration of 4 wt%, 7 wt%, 10 wt%, 14 wt%, or 15 wt%. In one embodiment, the anthocyanin concentration of an extract, for example a solid extract, is adjusted to a desired anthocyanin concentration by admixing a carrier such as maltodextrin or gum arabic. In one embodiment, a solid extract with an anthocyanin concentration of 15 wt% does not comprise an added carrier such as maltodextrin or gum arabic. In one embodiment, a solid extract with an anthocyanin concentration of 15 wt% is adjusted to obtain a solid extract having an anthocyanin concentration of 4 wt%, 7 wt%, 10 wt%, or 14 wt% by adding a carrier such as maltodextrin or gum arabic. In one embodiment, the term "added carrier such as maltodextrin or gum arabic" refers to a carrier such as maltodextrin or gum arabic added during the process of preparing said extract, preferably said carrier, e.g. maltodextrin or gum arabic, not being comprised in elderberry raw material. In one embodiment, a solid extract has a moisture content in the range of from 2.00 % to 8.00 %. In one embodiment, the solid extract comprises particles, wherein 98.0 - 100.0% of said particles have a particle size < 250 µm.

In one embodiment, a formulation of said extract, e.g. an extract in a solid form or an extract in a liquid form, further comprises, in addition to said extract, an excipient, e.g. a pharmaceutically acceptable excipient, a thickening agent, a sweetener, a sugar, a starch, a preservative, a flavoring agent, a nutritional oil such as a vegetable oil or fish oil, water, a milk such as a cow's milk, a soya milk, or an almond milk, an a black elderberry juice or other fruit juice, a vegetable juice, and/or a colorant.

In one embodiment, the term "healthy stage", as used herein, relates to a stage where a person is healthy and has not been infected with SARS-CoV-2. In a healthy stage, the patient has tested negative for a SARS-CoV2-infection, for example in a nucleic acid amplification assay testing for virus particles. In such healthy stage, the ultrafiltrated elderberry extract for use of the invention can be used to prevent an infection with SARS-CoV-2.

In one embodiment, the term "asymptomatic disease stage", as used herein, relates to a stage where a patient has tested positive for a SARS-CoV2-infection, for example in a nucleic acid amplification assay testing for virus particles, but has no symptoms of disease. In one embodiment, the term "mild or moderate disease stage", as used herein, relates to a disease stage, wherein a patient having a SARS-CoV2-infection shows one or several of mild or moderate symptoms, such as a mild throat pain, tiredness, fever, dry cough, loss of olfaction, headache. For example, an early moderate disease stage or moderate disease stage relates to a moderate illness in which individuals show evidence of lower respiratory disease during clinical assessment or imaging and in which individuals have an oxygen saturation (SpO₂) ≥ 94% on room air at sea level. In one embodiment, the extract for use is used to prevent a SARS-CoV2-infection, is used to prevent a SARS-CoV2-infection from progressing to a more severe disease stage, and/or is used to treat an early disease stage. In one embodiment, the term "severe disease stage", as used herein, relates to a disease stage, in which patients have Sp02 <94% on room air at sea level, a ratio of arterial partial pressure of oxygen to fraction of inspired oxygen (PaO₂/FiO₂) <300 mm Hg, respiratory frequency >30 breaths/min, or lung infiltrates >50%. In one embodiment, the term "critical disease stage", as used herein, relates to a disease stage, in which patients have respiratory failure, septic shock, and/or multiple organ dysfunction.

In one embodiment, the term "SARS-CoV-2 wildtype", as used herein, relates to severe acute respiratory syndrome coronavirus 2 (SARS□CoV□2) which is the virus that causes COVID-19 (coronavirus disease 2019), the respiratory illness responsible for the COVID-19 pandemic; preferably to the first sequenced SARS-CoV-2, as identified in Wuhan, China. First identified in the city of Wuhan, China, the World Health Organization declared the outbreak a Public Health Emergency of International Concern on 30 January 2020, and a pandemic on 11 March 2020. SARS□CoV□2 is a positive-sense single-stranded RNA virus that is contagious in humans. In one embodiment, the SARS-CoV-2 wildtype is severe acute respiratory syndrome coronavirus 2 isolate Wuhan-hu-1 or is SARS-CoV-2PR-1. In one embodiment, severe acute respiratory syndrome coronavirus 2 isolate Wuhan-hu-1 is as described in NCBI with accession number NC_045512.2. In one embodiment, severe acute respiratory syndrome coronavirus 2 isolate Wuhan-hu-1 has a sequence as defined in NCBI Reference Sequence NC_045512.2. In one embodiment, SARS-CoV-2PR-1 is as defined in [5].

In one embodiment, the term "SARS-CoV-2 variant", as used herein, relates to any variant of SARS-CoV-2 known to a person skilled in the art, e.g. an alpha variant, beta variant, gamma variant, or delta variant. In one embodiment, a SARS-CoV-2 variant is any of the variants of concern, variants of interest, or variants under investigation as declared by the World Health Organization, for example, the alpha, beta, gamma, delta, eta, iota, kappa, or lambda variant. In one embodiment, a SARS-CoV-2 variant is any of the variants of concern, as declared by the World Health Organization, for example, the alpha, beta, gamma, or delta variant. For example, the alpha variant relates to lineage B.1.1.7. The alpha variant emerged in the United Kingdom in September 2020. Notable mutations of the alpha variant include N501Y and P681H. The alpha variant may further include an E484K mutation. For example, the beta variant relates to lineage B.1.351. The beta variant emerged in South Africa in May 2020. Notable mutations of the beta variant include K417N, E484K, and N501Y. For example, the gamma variant relates to lineage P.1. The gamma variant emerged in Brazil in November 2020. Notable mutations also include K417N, E484K and N501Y. For example, the delta variant relates to lineage B.1.617.2. The delta variant emerged in India in October 2020. For example, the eta variant relates to lineage B.1.525. The eta variant has a E484K-mutation and a F888L mutation. For example, the iota variant relates to lineage B.1.526. For example, the kappa variant relates to a sublineage of lineage B.1.617, particularly relates to lineage B.1.617.1. For example, the lambda variant relates to lineage C.37.

In one embodiment, the SARS-CoV-2 variant is any variant selected from:alpha variant, beta variant, gamma variant, and delta variant, and/or is any variant selected from B.1.1.7 variant, e.g. with spike protein mutation E484K, 69del, 70del, 144del, N501Y, A570D, D614G, P681H, T716I, S982A, and/or D1118H;
B.1.351 variant, e.g. with spike protein mutation D80A, D215G, 241del, 242del, 243del, K417N, E484K, N501Y, D614G, and/or A701V;
P.1 variant, e.g. with spike protein mutation L18F, T20N, P26S, D138Y, R190S, K417T, E484K, N501Y, D614G, H655Y, and/or T1027I;
B.1.617 variant, e.g. with spike protein mutation L452R, E484Q, and/or D614G, such as B.1.617.1 variant, B.1.617.2 variant, or B.1.617.3 variant;
B.1.427 variant, e.g. with spike protein mutation L452R, and/or D614G;
B.1.429 variant, e.g. with spike protein mutation S13I, W152C, L452R, and/or D614G;
B.1.525 variant, e.g. with spike protein mutation A67V, 69del, 70del, 144del, E484K, D614G, Q677H, and/or F888L;
B.1.526 variant, e.g. with spike protein mutation T95I, D253G, and/or D614G; and
P.2 variant, e.g. with spike protein mutation E484K, D614G, and/or V1176F.

In one embodiment, said SARS-CoV-2 infection is an infection with any of SARS-CoV-2 wildtype, alpha variant, beta variant, gamma variant, or delta variant, e.g. any of B.1.1.7 variant, B.1.351 variant, P.1 variant, B.1.617 variant, B.1.617.1 variant, B.1.617.2 variant, B.1.617.3 variant, B.1.427 variant, B.1.429 variant, B.1.525 variant, B.1.526 variant, and P.2 variant. In a preferred embodiment, said SARS-CoV-2 infection is an infection with any of SARS-CoV-2 wildtype, alpha variant, beta variant, gamma variant, or delta variant, preferably SARS-CoV-2 wildtype, alpha variant, or beta variant; e.g. an infection with SARS-CoV-2 wildtype, SARS-CoV-2 alpha, e.g. B.1.1.7., or SARS-CoV-2 beta.

In one embodiment, the term "method of preparing an ultrafiltrated elderberry extract", as used herein, relates to a method of preparing an elderberry extract involving ultrafiltration. In one embodiment, said method of preparing an ultrafiltrated elderberry extract comprises a step of producing a juice of said one or more elderberries provided in step i), preferably prior to performing said ultrafiltration of step ii). In one embodiment, said method of preparing comprises a step of by juicing, mashing, grinding, shredding, chopping, pressing, blending, and/or pulping said one or more elderberries provided in step i), preferably prior to performing said ultrafiltration of step ii). In one embodiment, a retentate obtained from said ultrafiltration of step ii) is the ultrafiltrated elderberry extract. In one embodiment, said ultrafiltration enriches the anthocyanin content in said extract compared to an anthocyanin content of a raw material of said one or more elderberries provided in step i). In one embodiment, the one or more elderberries provided in step i) is elderberry raw material, e.g. elderberry fruits, such as fresh fruits or frozen fruits. In one embodiment, a retentate is obtained in step ii) by performing said ultrafiltration. In one embodiment, a retentate, which is obtained in step ii) by performing said ultrafiltration, comprises the high molecular weight fraction. In one embodiment, a retentate, which is obtained in step ii) by performing said ultrafiltration, comprises components that have a molecular weight which is larger than a molecular weight cut-off of at least 400 Da, preferably of at least 500 Da, more preferably of at least 1000 Da.

In one embodiment, said method of preparing an ultrafiltrated elderberry extract is a method of preparing a solid elderberry extract and/or a liquid elderberry extract. In one embodiment, said method of preparing an ultrafiltrated elderberry extract comprises a step of drying, preferably spray drying, said extract. In one embodiment, said method of preparing comprises a step of adding a carrier such as maltodextrin or gum arabic. In one embodiment, a method of preparing a solid elderberry extract comprises a step of spray drying a liquid elderberry extract, and optionally further comprises a step of adding a carrier such as maltodextrin or gum arabic. In one embodiment, said method of preparing an ultrafiltrated elderberry extract is a method of preparing an ultrafiltrated liquid elderberry extract, wherein said method optionally further comprises a step of drying said liquid extract, preferably spray drying said liquid extract, to obtain a solid extract.

In one embodiment, said one or more elderberries provided in step i) are elderberry raw material, particularly elderberry fruits, for example fresh elderberry fruits and/or frozen elderberry fruits. In one embodiment, the terms "elderberry" and "elderberry fruit" are used interchangeably. In one embodiment, the terms "elderberries" and "elderberry fruits" are used interchangeably. In one embodiment, when referring to "raw material" or "elderberry raw material", such term is relate to elderberry fruits prior to extraction and/or ultrafiltration.

In one embodiment, the one or more elderberries are subjected to a quality control prior to extraction and/or ultrafiltration. In one embodiment, the one or more elderberries provided in step i) are subjected to heating and/or thawing prior to step ii) of performing an ultrafiltration. In one embodiment, if the elderberry raw material is frozen, the frozen elderberry raw material is thawed prior to said ultrafiltration. In one embodiment, said one or more elderberries are heated to 30°C to facilitate enzymatic cell lysis. In one embodiment, the one or more elderberries provided in step i) are treated using one or more enzymes, e.g. a pectolytic enzyme, prior to said ultrafiltration of step ii). In one embodiment, the method of preparing ultrafiltrated elderberry extract comprises a step of mechanically, thermally, and/or enzymatically degrading said one or more elderberries provided in step i) prior to said ultrafiltration of step ii). In one embodiment, the method of preparing ultrafiltrated elderberry extract comprises a step of extracting said one or more elderberries, e.g., by juicing, mashing, grinding, shredding, chopping, pressing, blending, and/or pulping the elderberries, prior to said step ii) of performing ultrafiltration. In one embodiment, prior to said step ii) of performing ultrafiltration, the one or more elderberries and/or the elderberry extract are/is pasteurized and/or cooled. In one embodiment, the ultrafiltrated elderberry extract is aseptically filled into a container for storage.

In one embodiment, the method of preparing an ultrafiltrated elderberry extract comprises the steps:
1) providing one or more elderberries; wherein, preferably, said one or more elderberries comprise black elderberries; wherein, more preferably, said one or more elderberries comprise *Sambucus nigra L* and/or *Sambucus canadensis L*;
2) performing a water extraction;
3) performing an ultrafiltration using an ultrafiltration membrane having a molecular weight cut-off of at least 400 Da, preferably of at least 500 Da, more preferably of at least 1000 Da;
4) obtaining an ultrafiltrated elderberry extract.

In one embodiment, the term "water extraction", as used herein, relates to an extraction of said one or more elderberries, i.e. said elderberry raw material, using water, preferably by mixing said one or more elderberries with water and mechanically disrupting said one or more elderberries e.g. in a fruit press and/or under pressure. In one embodiment, said performing a water extraction comprises pressing said elderberries using water, preferably in a fruit press and/or under pressure. In one embodiment, the solvent in such water extraction is water. In one embodiment, by such water extraction, an extract, particularly water extract, is obtained in which the sugar content is reduced compared to the elderberry raw material, e.g. by washing out said sugar, and in which the anthocyanin content is maintained compared to the elderberry raw material. In one embodiment, the method of preparing an ultrafiltrated elderberry extract comprises a step of extracting said one or more elderberries using water, and a subsequent step of enriching anthocyanins and the high molecular weight fraction by ultrafiltration. In one embodiment, the ultrafiltration reduces the sugar content in the extract compared to the elderberry raw material. In one embodiment, by performing the ultrafiltration, the percentage of anthocyanins is increased in the ultrafiltrated extract compared to the elderberry raw material. In one embodiment, a water extract is obtained by mixing said one or more elderberries with water and mechanically disrupting said elderberries, e.g. by juicing, mashing, grinding, shredding, chopping, pressing, blending, and/or pulping the one or more elderberries, such as in a fruit press and/or under pressure. In one embodiment, the terms "degrading" and "disrupting" are used interchangeably. In one embodiment, the term "water extract" relates to an extract comprising extracted elderberries and water, preferably to a water extract comprising anthocyanins. In one embodiment, a water extract is an elderberry extract comprising anthocyanins and water. In one embodiment, a water extract is concentrated by ultrafiltration. In one embodiment, the term "water extract" relates to an extract that has not (yet) been subjected to ultrafiltration. In one embodiment, anthocyanins and a high molecular weight fraction becomes enriched in an extract by ultrafiltration.

In one embodiment, a liquid elderberry extract is dried, e.g. by spray drying, to obtain a solid elderberry extract. In one embodiment, the method of preparing ultrafiltrated elderberry extract comprises drying, e.g. by spray drying and/or lyophilization, said ultrafiltrated elderberry extract obtained in step iii). In one embodiment, the method of preparing ultrafiltrated elderberry extract comprises adding a carrier such as maltodextrin or gum arabic to said ultrafiltrated elderberry extract. The method of preparing an ultrafiltrated elderberry extract is highly advantageous in that no organic solvent, e.g. ethanol, is needed. The method is advantageous in that it is characterized by physical extraction. In one embodiment, an ultrafiltrated extract is dried using a drying method, for example, spray drying and/or lyophilization, preferably spray drying. In one embodiment, after said step of performing a spry drying, the ultrafiltrated elderberry extract, e.g. ultrafiltrated elderberry extract powder, is sieved, preferably with a cut-off of 0.5 mm. In one embodiment, the method of preparing a dried ultrafiltratedelderberry extract further comprises admixing said ultrafiltrated elderberry extract with a carrier such as maltodextrin or gum arabic. For example, prior to a spray drying, a carrier such as maltodextrin or gum arabic is added, e.g. to obtain an ultrafiltrated elderberry extract in the form of a powder, e.g. a 4 wt%, 7 wt%, 10 wt%, or 14 wt% anthocyanin ultrafiltrated elderberry extract.

In one embodiment, the method of preparing an ultrafiltrated elderberry extract comprises:
a) providing one or more elderberries; wherein, preferably, said one or more elderberries comprise black elderberries; wherein, more preferably, said one or more elderberries comprise *Sambucus nigra* and/or *Sambucus canadensis*;
b) optionally, heating and/or thawing said one or more elderberries;
c) optionally, treating said one or more elderberries with an enzyme, preferably a pectolytic enzyme;
d) extracting anthocyanins from said one or more elderberries, preferably using a water extraction, e.g. by adding water and mechanically disrupting said one or more elderberries such as by juicing, mashing, grinding, shredding, chopping, pressing, blending, and/or pulping the one or more elderberries, to obtain a water extract comprising anthocyanins;
e) optionally, pasteurizing and/or cooling said water extract;
f) enriching the anthocyanins in said water extract by performing an ultrafiltration using an ultrafiltration membrane having a molecular weight cut-off of at least 400 Da, preferably of at least 500 Da, more preferably of at least 1000 Da;
g) optionally, pasteurizing and/or cooling;
h) obtaining an ultrafiltrated elderberry extract, e.g. a liquid ultrafiltrated elderberry extract.

In one embodiment, the method of preparing ultrafiltrated elderberry extract further comprises:
i) mixing the ultrafiltrated elderberry extract obtained in step h), e.g. a liquid ultrafiltrated elderberry extract obtained in step h), with a carrier such as maltodextrin or gum arabic, thereby obtaining an extract mixture;
j) optionally, drying said extract mixture, e.g. by spray drying;
k) optionally, sieving said extract mixture obtained in step i) or, if step j) is present, said dried extract mixture obtained in step j), preferably with a cut-off 0.5 mm;
l) obtaining an ultrafiltrated elderberry extract, e.g. a solid ultrafiltrated elderberry extract.

In one embodiment, a concentration of a component of the elderberry extract, e.g. an anthocyanin concentration, is measured using HPLC. In one embodiment, an anthocyanin concentration is determined using HPLC.

In one embodiment, the elderberry extract comprises an excipient, preferably maltodextrin or gum arabic. In one embodiment, a method of preparing an ultrafiltrated elderberry extract comprises adding an excipient, preferably maltodextrin or gum arabic, to the ultrafiltrated elderberry extract. In one embodiment, an excipient, preferably maltodextrin or gum arabic, is added to an ultrafiltrated elderberry extract, preferably to an extract in the form of a powder. In one embodiment, an ultrafiltrated elderberry extract in the form of a powder comprises an excipient, preferably maltodextrin or gum arabic. In one embodiment, an excipient is a carrier such as maltodextrin or gum arabic.

When used herein, the term "ElderCraft^{®}" relates to an exemplary ultrafiltrated elderberry extract of the invention. In one embodiment, the elderberry extract for use of the invention is an ElderCraft^{®} extract, e.g. an ElderCraft^{®} 3.2% Liquid Extract, ElderCraft^{®} 4% Extract Powder, ElderCraft^{®} 7% Extract Powder, ElderCraft^{®} 10% Extract Powder, ElderCraft^{®} 14% Extract Powder, or ElderCraft^{®} 15% Extract Powder. For example, an elderberry extract for use of the invention is a black elderberry liquid standardized to a minimum anthocyanin content of 3.2% via an ultrafiltration extraction which enriches active compounds without harsh chemical solvents, such as an ElderCraft^{®} 3.2% Liquid Extract, or a spray-dried black elderberry extract standardized to a minimum anthocyanin content of e.g. 4% via an ultrafiltration extraction which enriches active compounds without harsh chemical solvents, such as ElderCraft^{®} 4% Extract Powder, ElderCraft^{®} 7% Extract Powder, ElderCraft^{®} 10% Extract Powder, ElderCraft^{®} 14% Extract Powder, or ElderCraft^{®} 15% Extract Powder. In one embodiment, a composition of the invention comprises or consists of an ElderCraft^{®} extract, e.g. an ElderCraft^{®} 3.2% Liquid Extract, ElderCraft^{®} 4% Extract Powder, ElderCraft^{®} 7% Extract Powder, ElderCraft^{®} 10% Extract Powder, ElderCraft^{®} 14% Extract Powder, or ElderCraft^{®} 15% Extract Powder.

In one embodiment, an ultrafiltrated elderberry extract is advantageous over an elderberry ethanol extract in that it comprises higher amounts of polysaccharides, vitamins, and/or minerals. As demonstrated herein, the polysaccharides present in the ultrafiltrated elderberry extract have a highly efficient immunostimulatory effect and are effective against SARS-CoV2. Advantageously, the ultrafiltrated extract of the invention is not only highly effective against SARS-CoV2 wildtype, but also against SARS-CoV2 variants, such as the SARS-CoV2 alpha variant and the SARS-CoV2 beta variant. The ultrafiltrated elderberry extract for use of the invention is highly effective in inhibiting viral replication of SARS-CoV2. Unexpectedly, the ultrafiltrated elderberry extract for use does not only inhibit viral replication of SARS-CoV2 wildtype, but also of SARS-CoV2 variants such as the alpha and beta variant. The ultrafiltrated extract for use of the invention is highly efficient in preventing and treating SARS-CoV2 infections, e.g. infections with SARS-CoV2 wildtype, alpha variant, or beta variant.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

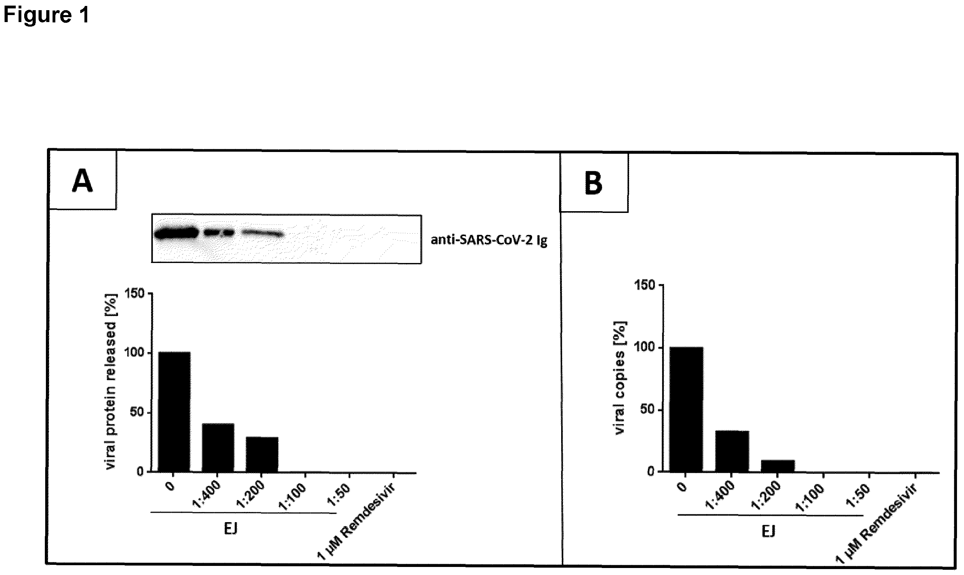

**Figure 1** shows that elderberry juice (EJ) is effective against SARS-CoV-2. Elderberry juice was tested with regard to an inhibition of SARS-CoV-2 in Vero-B4 cells. Remdesivir was used as a control. As shown in Figure 1 A), elderberry extract is effective in inhibiting viral protein release. Particularly, at a dilution of 1:200, the elderberry juice significantly decreases viral protein release. At a dilution of 1:100, viral protein release is effectively inhibited, as no viral protein release is measurable. It was found that elderberry juice (EJ) inhibits SARS-CoV-2 replication in Vero B4 cells. (A) Western Blot analysis of released virions. Supernatants were harvested 3 days post infection (dpi), virions were purified and analyzed by Western Blot using antibodies derived from convalescent SARS-CoV-2 patient sera. Densitometric analysis using AIDA^{®}, one representative Western Blot is shown. (B) qRT-PCR of cell culture supernatants after 3 days of treatment with EJ.

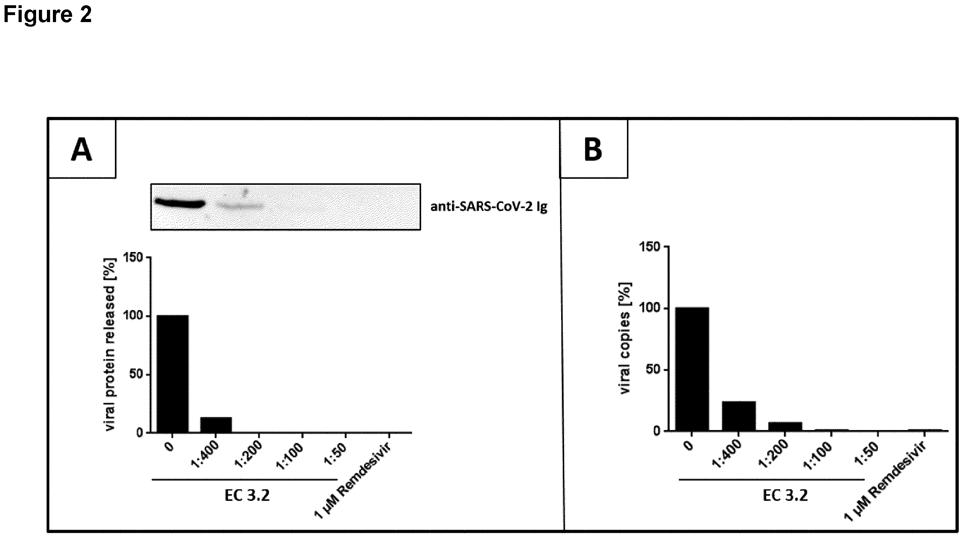

**Figure 2** shows that elderberry extract is highly effective against SARS-CoV-2. The ultrafiltrated elderberry extract of the invention, particularly an ultrafiltrated elderberry extract comprising 3.2 wt% anthocyanin (EC3.2), was tested with regard to an inhibition of SARS-CoV-2 in Vero-B4 cells. Remdesivir was used as a control. As shown in Figure 2 A), elderberry extract is highly effective in inhibiting viral protein release. Particularly, at a dilution of 1: 400, the elderberry extract significantly decreases viral protein release. At a dilution of 1:200, viral protein release is effectively inhibited, as no viral protein release is measurable. Ultrafiltrated elderberry extract is more effective in inhibiting a SARS-CoV-2 infection than elderberry juice. It was found that ultrafiltrated elderberry extract, e.g. EC 3.2, is highly efficient in inhibiting SARS-CoV-2 replication in Vero B4 cells. (A) Western Blot analysis of released virions. Supernatants were harvested 3 days post infection (dpi), virions were purified and analyzed by Western Blot using antibodies derived from convalescent SARS-CoV-2 patient sera. Densitometric analysis using AIDA^{®}, one representative Western Blot is shown. (B) qRT-PCR of cell culture supernatants after 3 days of treatment with ultrafiltrated elderberry extract.

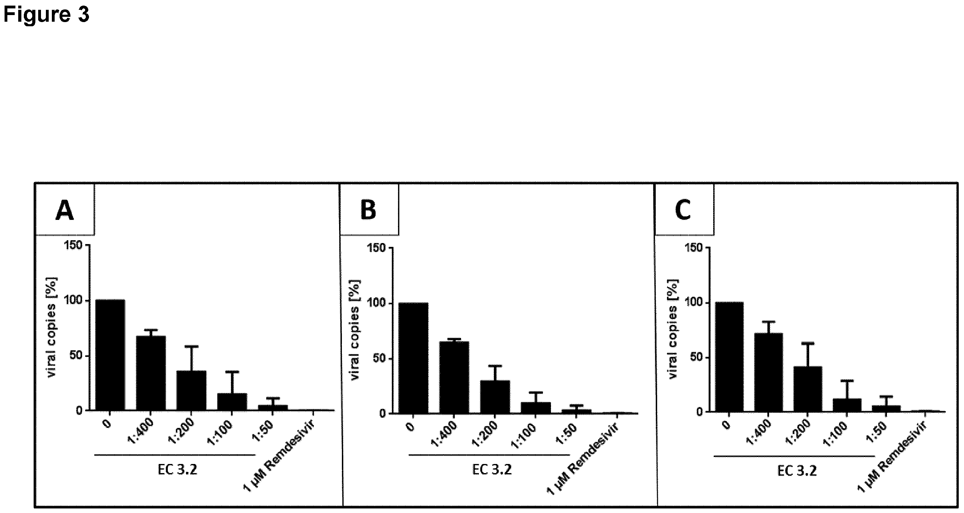

**Figure 3** shows that an ultrafiltrated elderberry extract of the invention effectively inhibits viral replication of SARS-CoV-2, particularly SARS-CoV-2 variants such as alpha- and beta-variants. Ultrafiltrated elderberry extract is highly effective in inhibiting viral replication of the tested SARS-CoV-variants: A) SARS-CoV-2 wildtype, B) SARS-CoV-2 Alpha and C) SARS-CoV-2 Beta. The experiments were performed using A549-cells expressing ACE2 and TMPRSS2. It was found that ultrafiltrated elderberry extract, e.g. EC 3.2, inhibits replication of SARS-CoV-2_{PR-1} wt, Alpha and Beta in human A549 ACE2/TMPRSS2+ cells with comparable efficacy. qRT-PCR analysis of cell culture supernatants after 3 days of treatment with ultrafiltrated elderberry extract. A549 ACE2/TMPRSS2+ cells were infected with either SARS-CoV-2_{PR-1} wt (A), Alpha (B) or Beta (C) and supernatants were harvested 3 dpi. Bars show analysis of three independent experiments +/- SD.

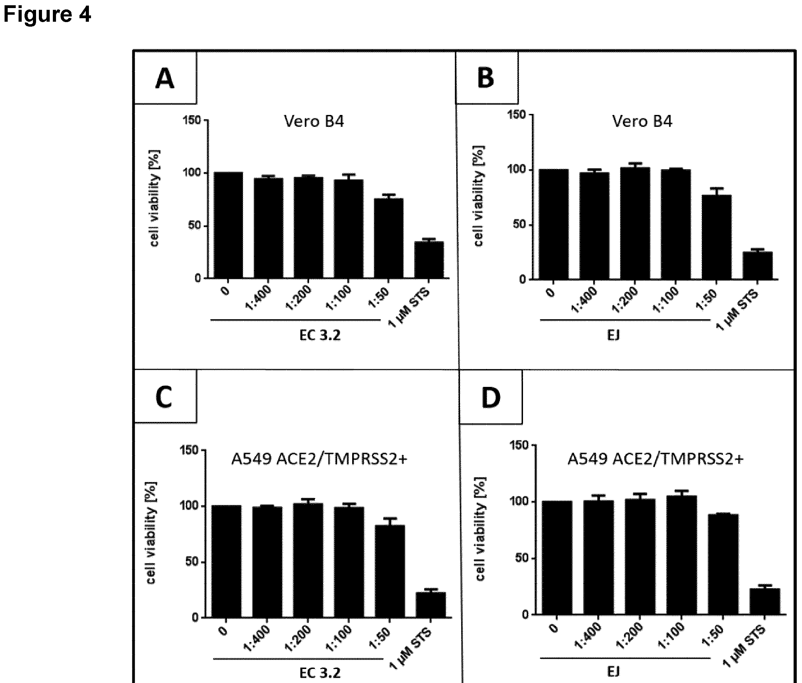

**Figure 4** shows that neither elderberry juice (EJ) nor elderberry extract (EC3.2) decreases cell viability of Vero B4 and A549 ACE2/TMPRSS2+ cells. Cell viability of Vero-B4 cells was analyzed by WST assay. The ultrafiltrated extract of the invention (e.g. ElderCraft^{®}) does not reduce cell viability of the cells. Vero B4 (A and B) or A549 ACE2/TMPRSS2+ (C and D) cells were incubated with indicated concentrations of EC 3.2 (A and C) or EJ (B and D). As a positive control, Staurosporine (STS) as an inducer of apoptosis was added at a concentration of 10 µM. Following treatment for three days, the cell viability was measured by WST-1 assays. Bars show mean values of three independent experiments +/- SD.

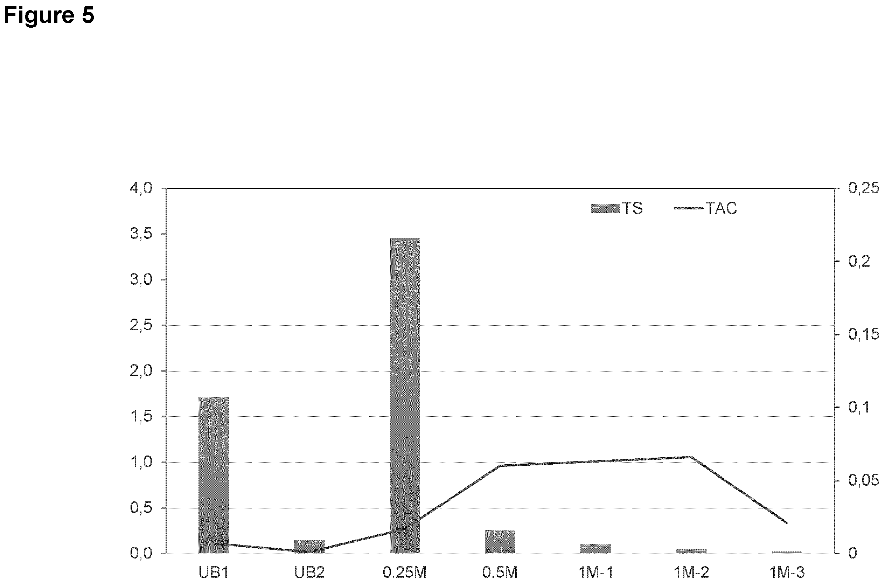

**Figure 5** shows the elution profile of the purification step on an ion exchange DEAE-Sepharose column. Columns represent total sugars (TS), and the line represents anthocyanin content (TAC=total anthocyanin content). Flow-through fractions UB1 and UB2 were combined and labeled as sample "UNBOUND" and Fraction 0.25 M was labeld as BOUND and used for further analysis. Samples were assessed for anthocyanin content and polysaccharide content using the phenol sulphuric acid method. The analysis shows that it was possible to enrich polysaccharides in fraction Bound and Unbound, and separate them from the anthocyanin content, additionally. Neutral polysaccharides are likely in the fraction UNBOUND.

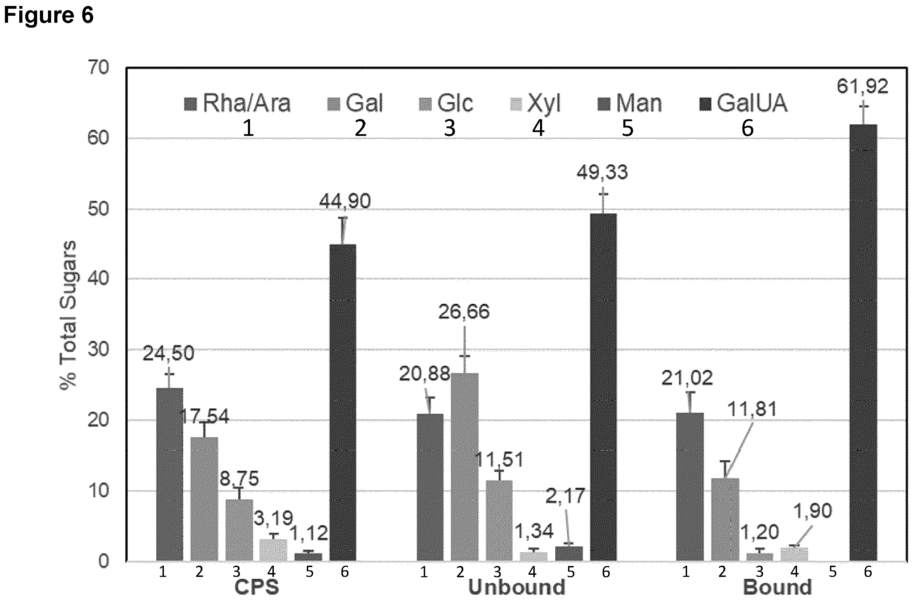

**Figure 6** shows a monosaccharide composition of a crude polysaccharide sample (CPS), an unbound fraction (UNBOUND) and a bound fraction (BOUND). The figure represents represent averages of % of total monosaccharide content ± SE, n = 4 for CPS and 6 for unbound and bound samples. The bound fraction was enriched in galacturonic acid and contained galactose, arabinose and rhamnose. The BOUND sample had notably reduced amounts of glucose, galactose, xylose and mannose compared to the CPS, which indicated the removal of neutral polysaccharides by the ion exchange procedure.

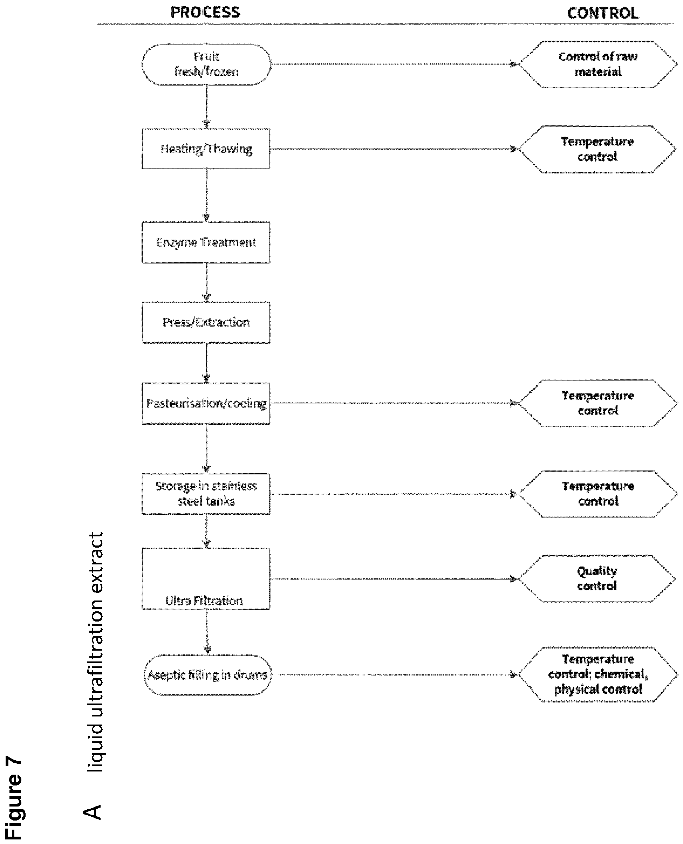

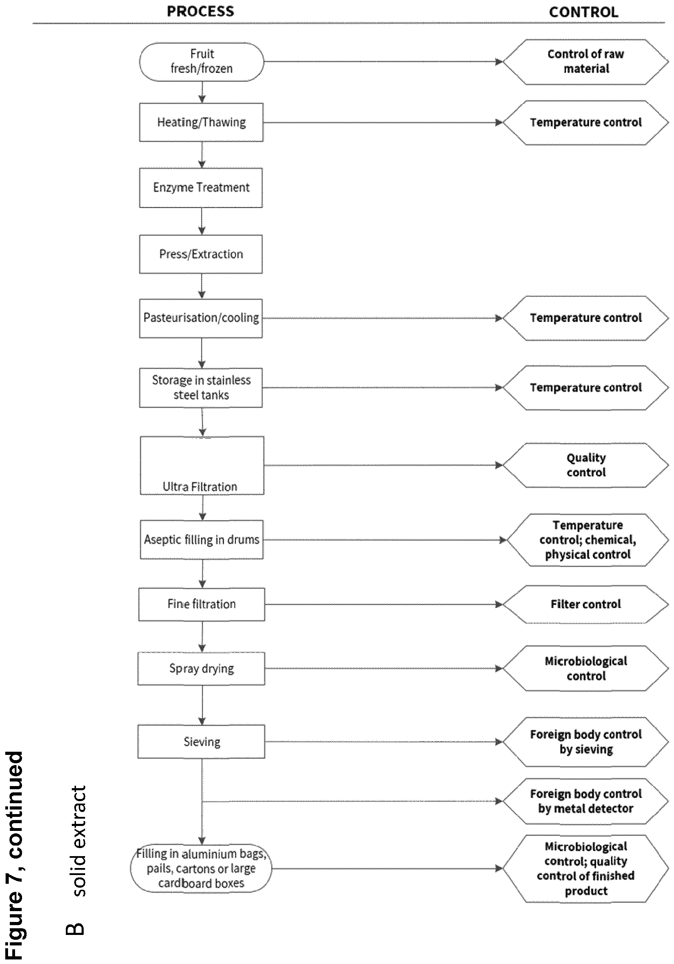

**Figure 7** shows a schematic flowchart of an exemplary method of preparing an ultrafiltrated elderberry extract, e.g. an ultrafiltrated elderberry extract in a liquid form (A) or in a solid form (B).

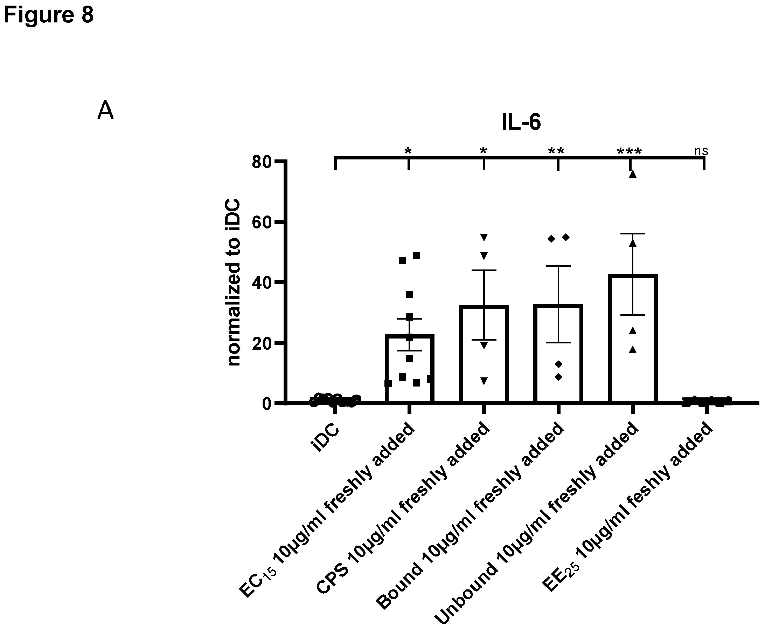

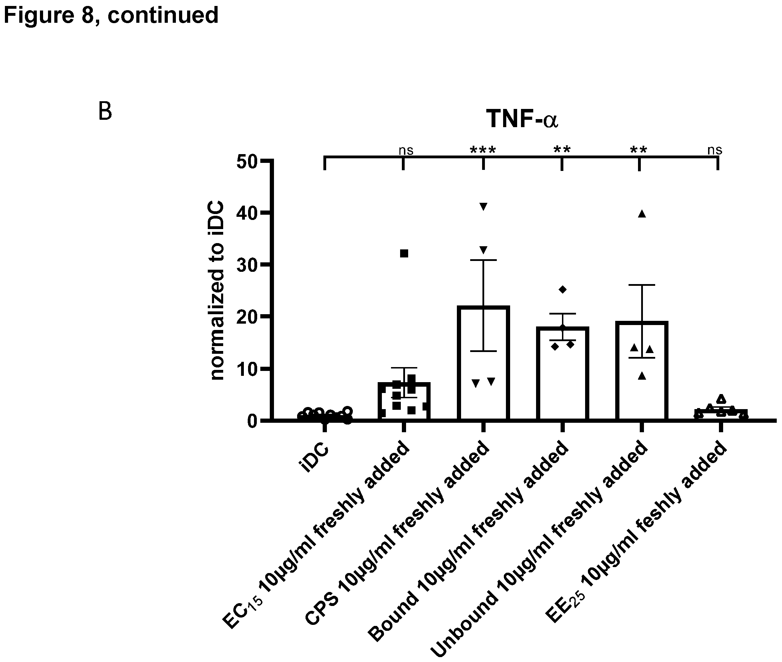

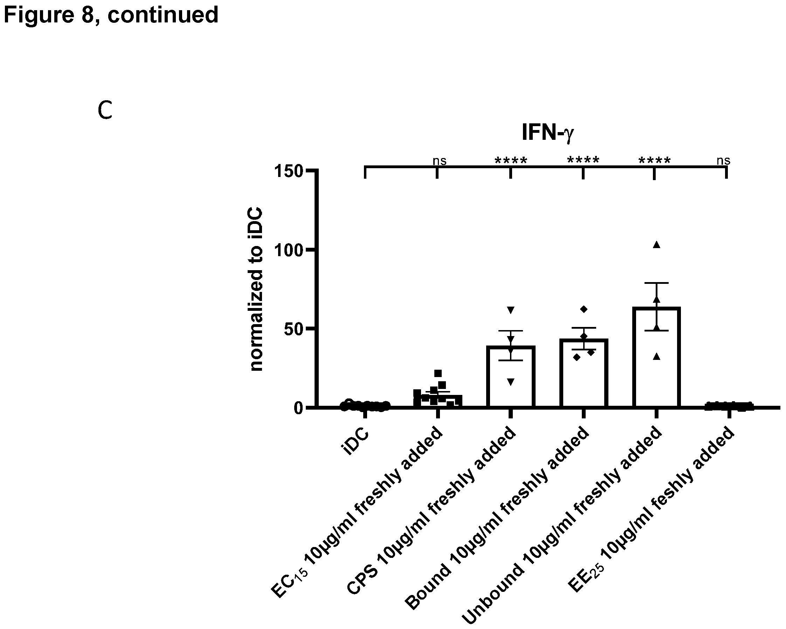

**Figure 8** shows cytokine secretion.
   A) shows the cytokine secretion of IL-6 into the supernatant of mixed lymphocyte reaction (MLR) co-cultures.
      EC15 induced a significant increased IL-6 release into the supernatant, when compared with untreated iDCs. A very strong and statistically significant increase in cytokine secretion was induced when the cells were incubated with the polysaccharide rich fractions CPS, Bound or Unbound fractions, respectively. In sharp contrast, EE25 induced no significant IL-6 secretion, when compared to iDCs. Thus, these data clearly indicate that elderberry extract EC15, and especially fractions CPS, Bound and Unbound, induce highly elevated cytokine expression levels, when compared to untreated control iDCs. On day 7, human pre-treated DCs were co-cultured at different ratios with 4^{∗}10⁵ allogeneic NAF cells, and substances were added again at the same concentration as used on day 6. In the control setting, untreated DCs were also co-cultured at different ratios with 4^{∗}10⁵ allogeneic NAF cells, without the addition of Elderberry derived substances. Statistical analyses were performed using the one-way ANOVA (n = 4).
   B) shows the cytokine secretion of TNF-a into the supernatant of MLR co-cultures. Cytokine secretion of DC co-cultures in the presence of elderberry extract and its fractions was analysed. EC15 slightly induced TNF-a secretion, in comparison to untreated control iDCs, while fractions CPS, Bound and Unbound, induced statistically increased cytokine levels, in comparison to untreated control iDCs. In sharp contrast, EE25 induced no significant TNF-a secretion, when compared to iDCs. In the fractions CPS, Bound and Unbound, the immunostimulatory substances, particularly polysaccharides, are enriched compared to the extract; thus, the immunostimulatory effect of the extract fractions is even further enhanced compared to the immunostimulatory effect of the extract. On day 7, human pre-treated DCs were co-cultured at different ratios with 4^{∗}10⁵ allogeneic NAF cells, and substances were added again at the same concentration as used on day 6. In the control setting, untreated DCs were also co-cultured at different ratios with 4^{∗}10⁵ allogeneic NAF cells, without the addition of Elderberry derived substances. Statistical analyses were performed using the one-way ANOVA (n = 4).
   C) shows the cytokine secretion of IFN gamma into the supernatant of MLR co-cultures. Cytokine secretion of DC co-cultures in the presence of elderberry extract and its fractions was analyzed. Fractions CPS, Bound and Unbound, induced statistically highly significant increased IFN-gamma levels, in comparison to EC15 or untreated control iDCs. In sharp contrast, EE25 induced no significant IFN-gamma secretion, when compared to iDCs.

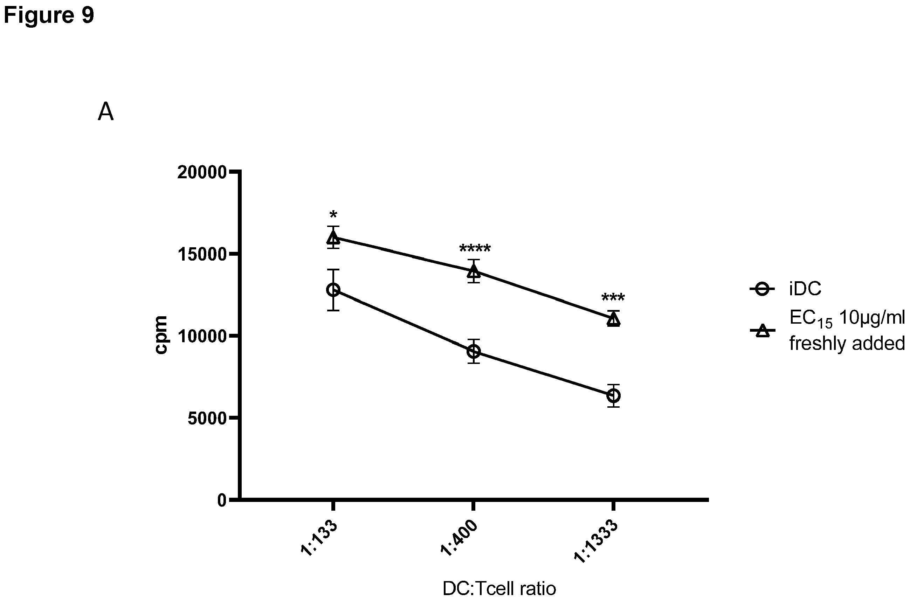

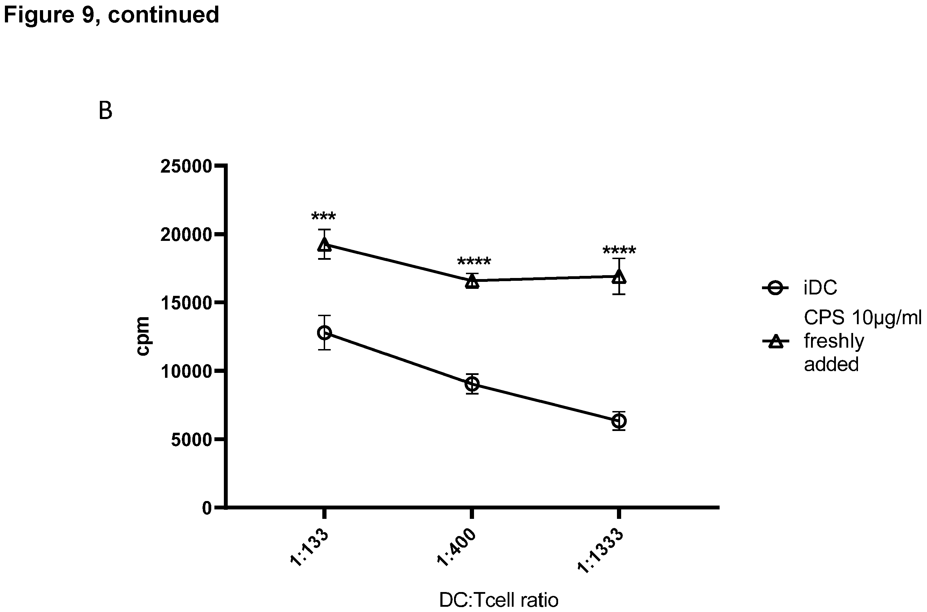

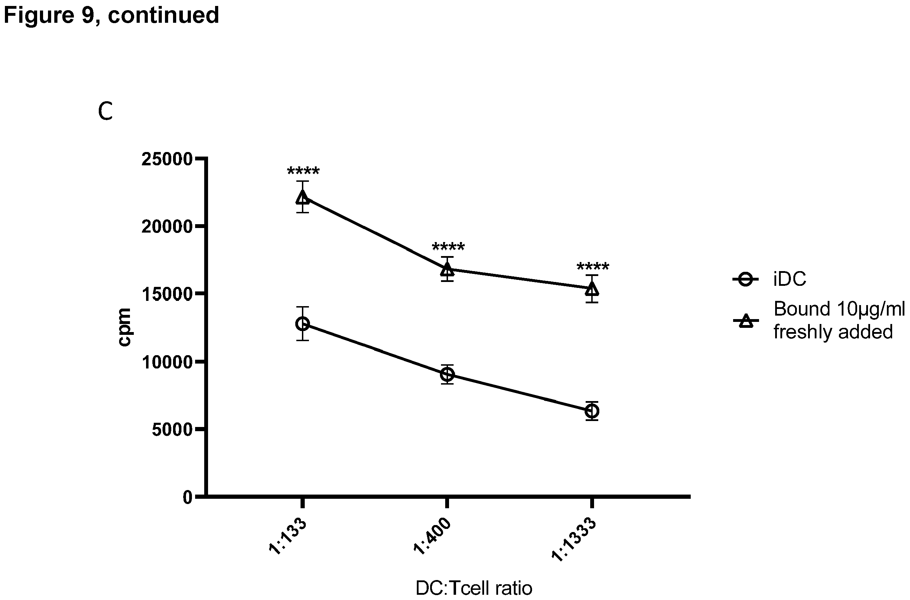

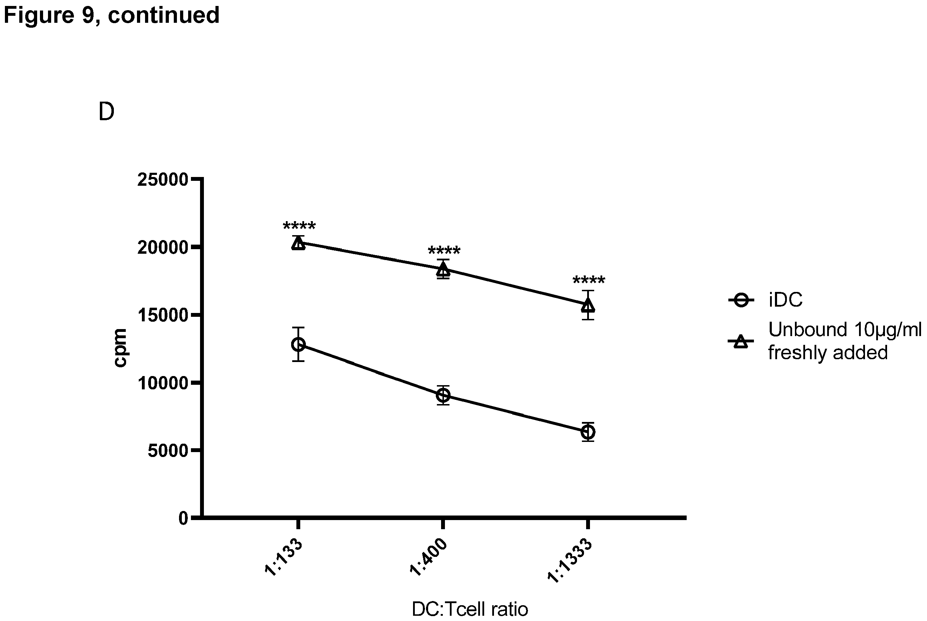

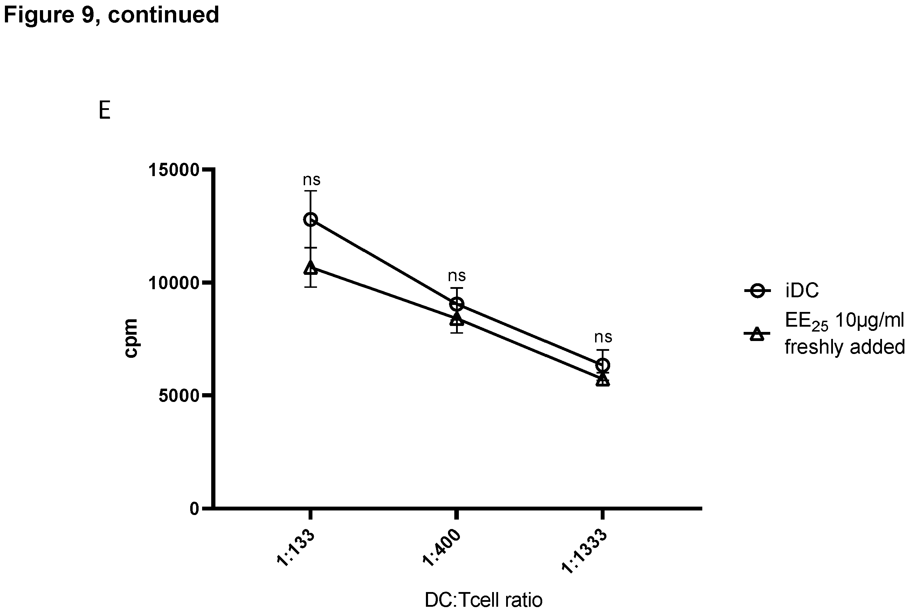

**Figure 9** shows that iDCs induce only a week T cell proliferative response. A) EC15 induced a significantly stronger T cells proliferation. B-D) The fractions CPS, Bound and Unbound induced an even higher T cell response, when compared to untreated control iDCs. E) EE25 did not induce an increased T cell response, when compared to untreated control iDCs. Thus, DCs incubated with EC15, CPS, Bound and Unbound induce a very potent T cell specific immune response. It was found that dendritic cells incubated with the fractions EC15, CPS, Bound and Unbound induced a potent T cell proliferation in MLR co-cultures. To analyze the T cell stimulatory capacity of DCs treated with EC15, EE25 and ultrafiltrated elderberry extract and fractions thereof, cells were pulsed with 3H-thymidine (1 µC/well; PerkinElmer, Rodgau, Germany) for 16 - 20 h. Thymidine incorporation was assessed using a Wallac 1420 Victor2 Microplate Reader (PerkinElmer). Statistical analyses were performed using the two-way ANOVA (n = 3).

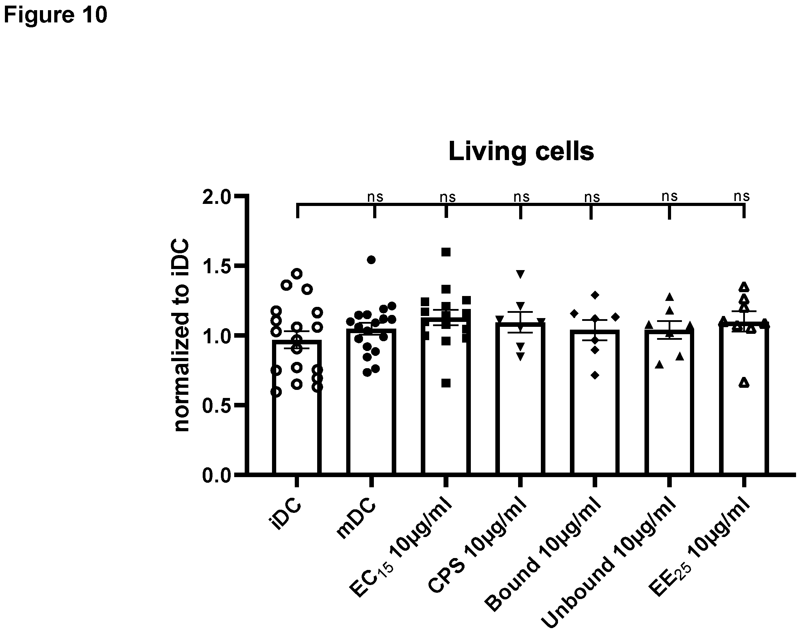

**Figure 10** shows that none of the analyzed Elderberry extracts and fractions, i.e EC15, EE25, CPS, Bound and Unbound, induced significant levels of cell death, when compared to untreated control iDCs. For dead cell discrimination, 7-AAD viability staining solution (ThermoFisherScientific) was added directly to the surface staining, prior to subsequent FACS analyses.

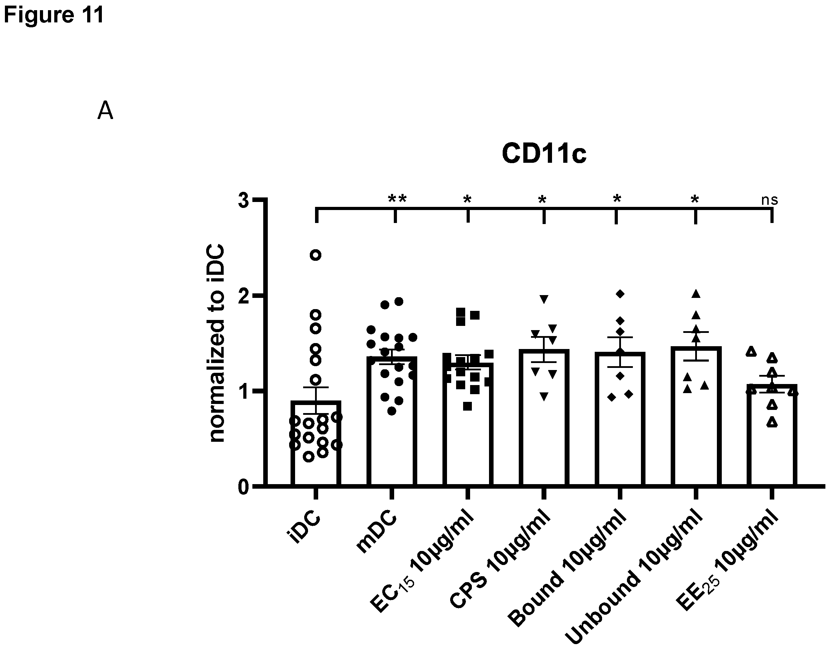

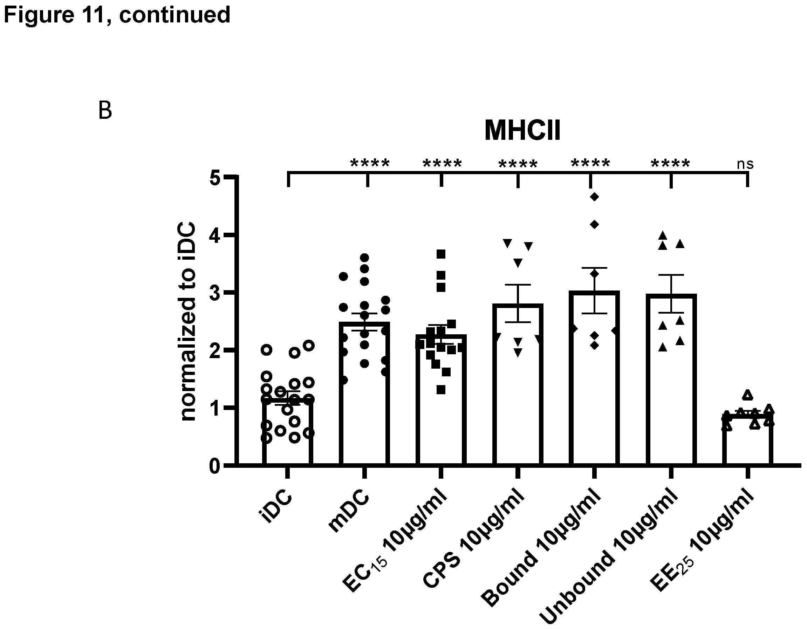

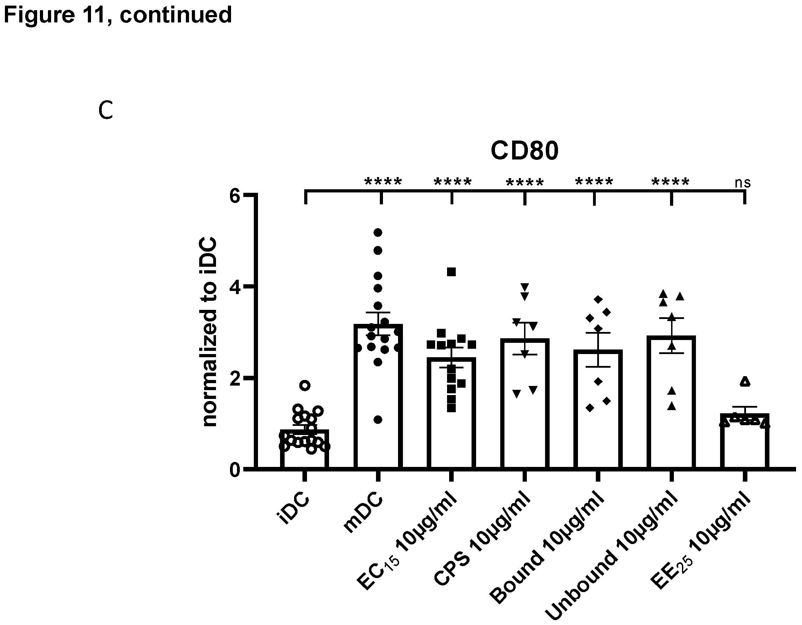

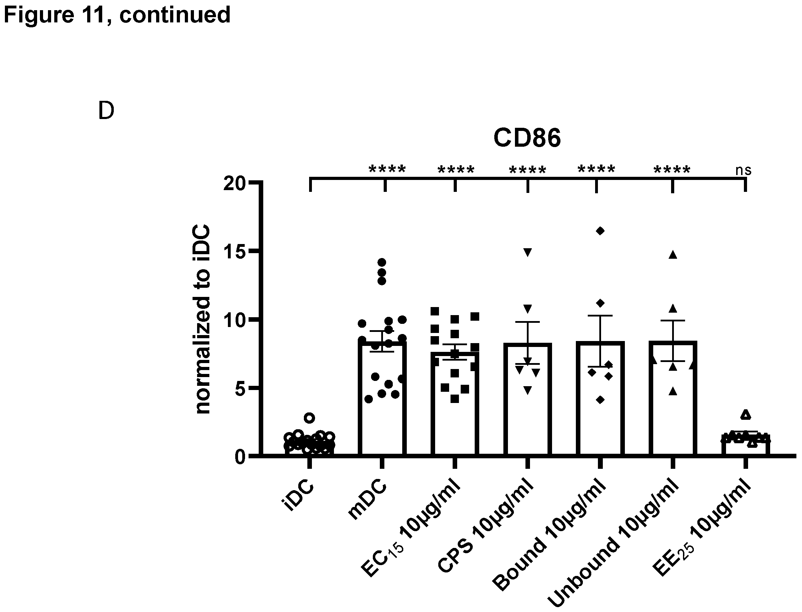

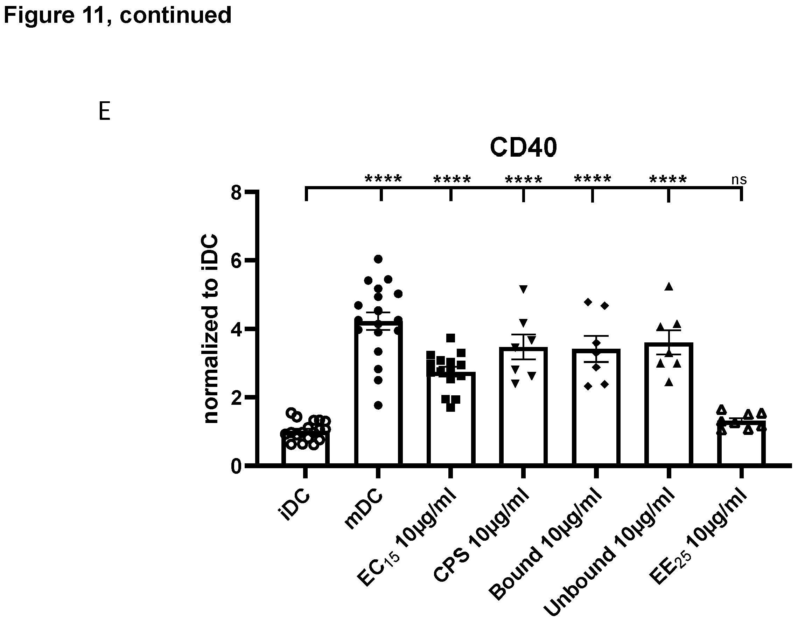

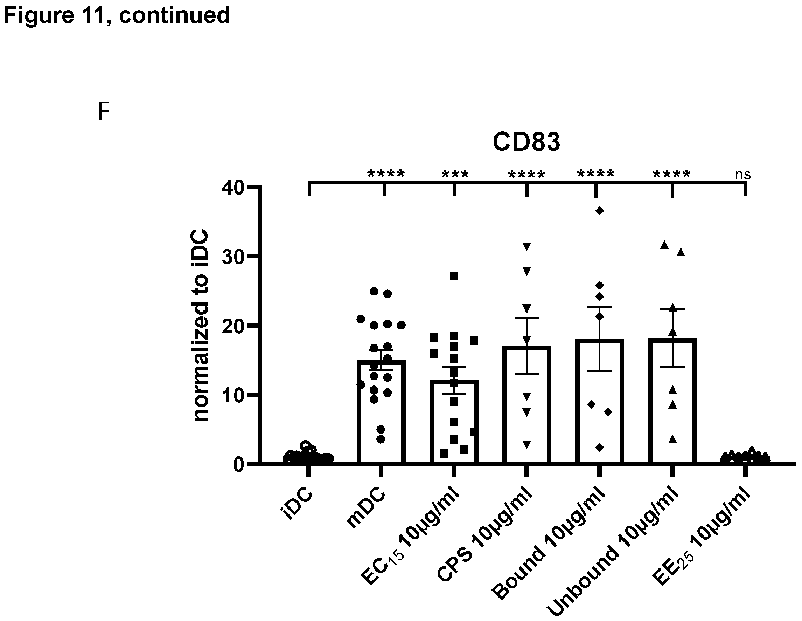

**Figure 11** shows the phenotypical characterization of human iDCs which were incubated with the Elderberry extracts and fractions, EC15, EE25, CPS, Bound and Unbound. The following monoclonal antibodies were used for phenotypic FACS analyzes (all purchased from BioLegend, unless stated otherwise): CD11c-APC (clone 3.9), CD40-PECy7 (clone 5C3), CD80-BV421 (clone 2D10) CD83-PE (clone HB15e), CD86-FITC (clone FUN-1; BD) and MHCII-APCCy (clone L243). The surface staining was performed for 30 min at 4°C in the dark in DPBS, prior to the subsequent FACS analyses. A-F) show that all Elderberry extracts and fractions, except EE25, induces a statistically significant expression of CD11c, MHCII, CD80, CD86, CD40 and CD83, when compared to untreated control iDCs. LPS-matured DC (mDC) were used as a positive control. Thus, except EE25, all analyzed Elderberry extracts and fractions induced a significant DC maturation phenotype, which correlates very well with the observed increased cytokine secretion levels (see Figure 8A-C) as well as with the enhanced DC-mediated T cell proliferation (see Figure 9A-E).

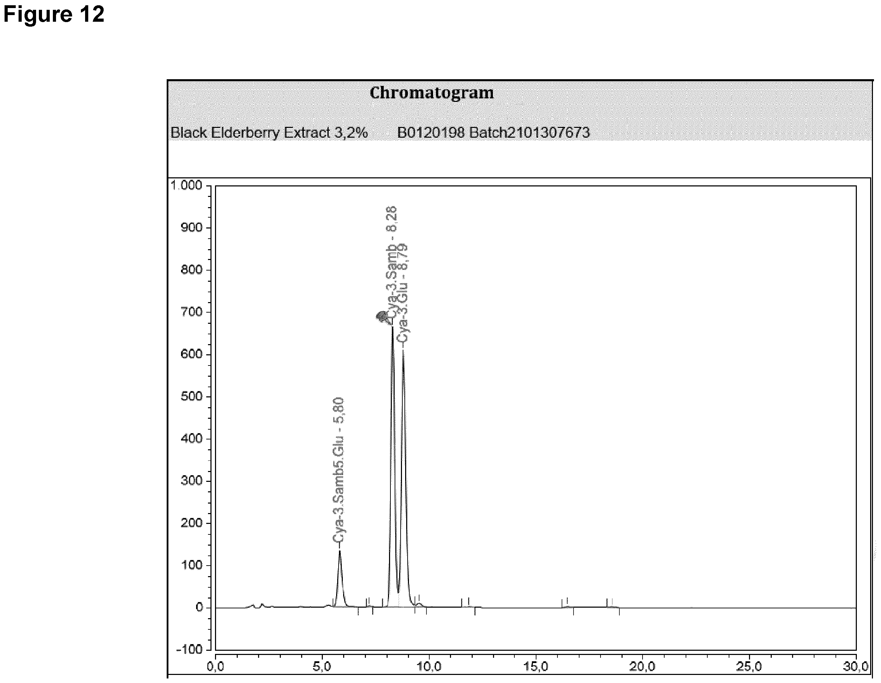

**Figure 12** shows a HPLC chromatogram of an anthocyanin analysis of an ultrafiltrated elderberry extract. Three independent peaks can be identified corresponding to the anthocyanins cyanidin-3-sambubioside-5-glucoside (peak 1), cyanidin 3-sambubioside (peak 2) and cyanidin-3-glucoside (peak 3). A total content of content of 34.1 g/kg anthocyanin expressed as cyanidin 3-glucoside was determined.

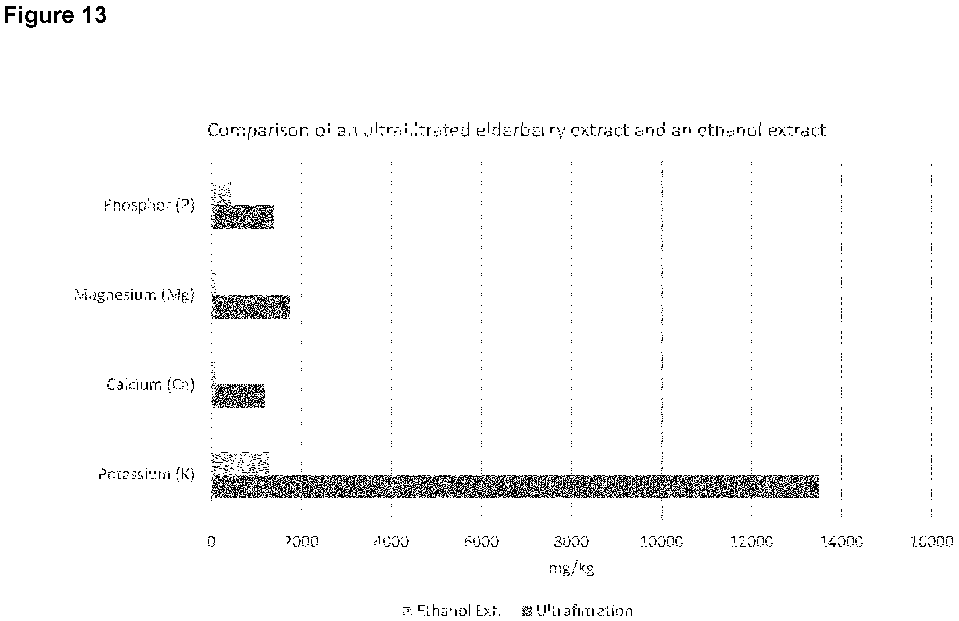

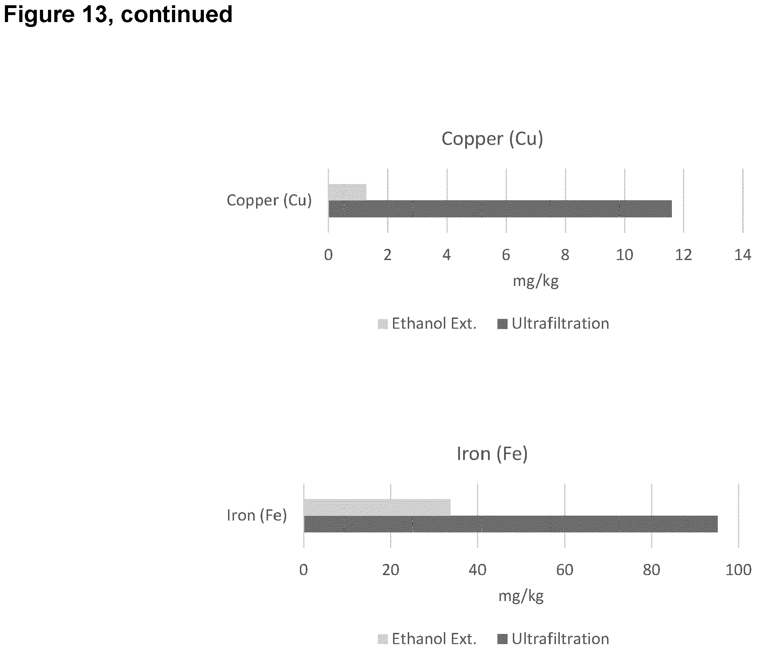

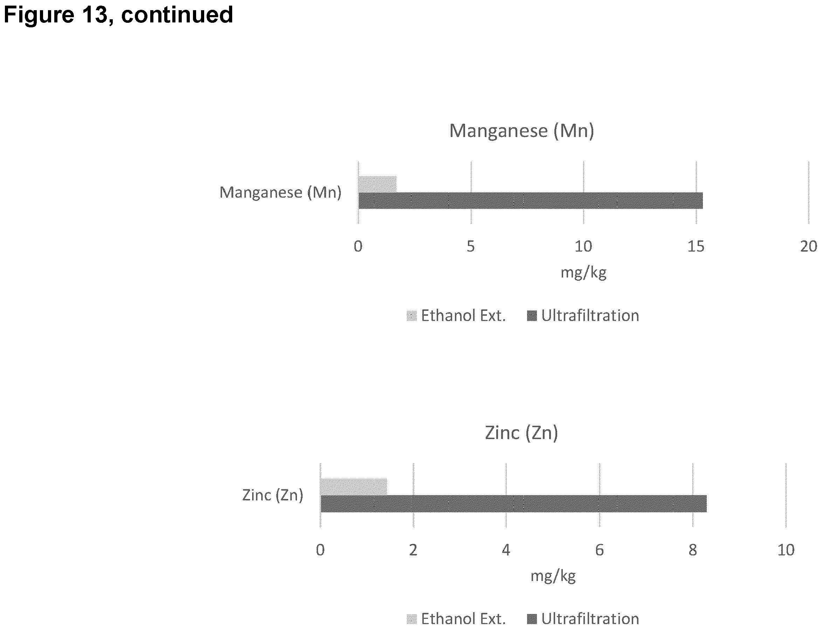

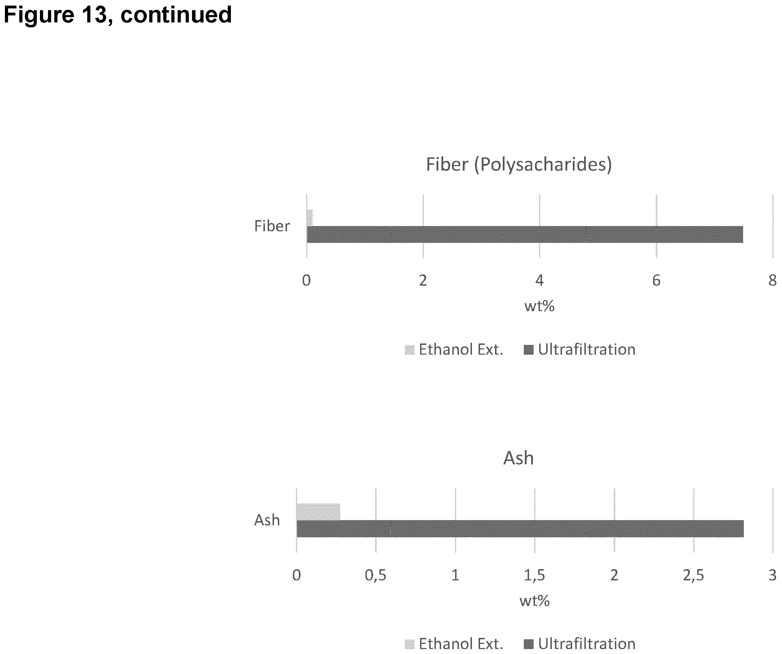

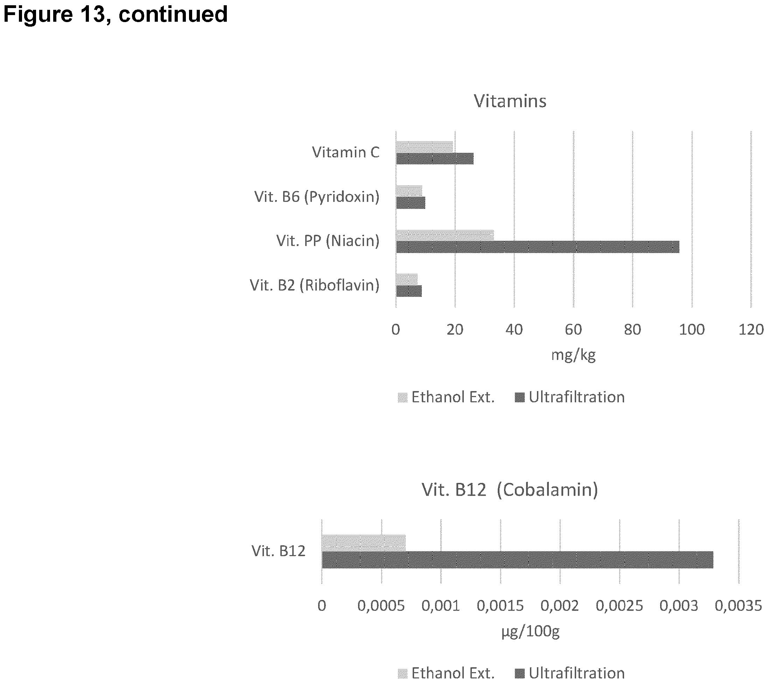

**Figure 13** shows a comparison of an exemplary ultrafiltrated elderberry extract of the invention and a state of the art ethanol extract. The here described extraction and ultrafiltration method is beneficial in providing a higher amounts of phosphor, magnesium, calcium, potassium, copper, iron, manganese, zinc, and fiber (polysaccharides).

In the following, reference is made to the examples, which are given to illustrate, not to limit the present invention.

### EXAMPLES

### Example 1: Preparation of ultrafiltrated elderberry extract.

The flowchart of a method of preparing ultrafiltrated elderberry extract is shown in figure 7. The elderberry extract is prepared by providing elderberry fruits, i.e. elderberry raw material, for example fresh elderberry fruit and/or frozen elderberry fruits. Optionally, the elderberry fruits are subjected to a quality control. Optionally, the elderberry fruits are subjected to heating and/or thawing. The elderberry fruits are extracted, e.g., by juicing, mashing, grinding, shredding, chopping, pressing, blending, and/or pulping the fruit raw material to obtain an elderberry extract. Optionally, the elderberry extract is pasteurized and/or cooled. The elderberry extract is then ultrafiltrated to obtain an ultrafiltrated elderberry extract (Fig. 7A). The ultrafiltration enriches anthocyanins in said extract and allows to standardize the extract to a defined anthocyanin concentration. Optionally, the ultrafiltrated elderberry extract is subsequently aseptically filled into a container for storage. The thus obtained ultrafiltrated elderberry extract is a liquid elderberry extract. Optionally, the liquid elderberry extract can be subsequently dried, e.g. by spray drying and/or lyophilization, to obtain a solid elderberry extract. Optionally, a carrier, such as maltodextrin or gum arabic, is added to such solid elderberry extract. The method provides a stable extract having a standardized amount of anthocyanins and polyphenols. The method of preparing ultrafiltrated elderberry extract is highly advantageous in that the method comprises physical extraction without the use of organic solvents. The method is advantageous in that it only comprises physical extraction. Organic solvents, such as ethanol, are not needed. The method is further advantageous in that the extract comprises the important nutrients and minerals that are comprised in the elderberry raw material. The amount of monosaccharides and disaccharides, as well as salt and water, is typically reduced in said extract compared to said elderberry raw material. Furthermore, the method of preparing the elderberry extract of the invention is highly advantageous in that anthocyanins are enriched in the extract obtained using ultrafiltration.

### ULTRAFILTRATED ELDERBERRY EXTRACT IN A LIQUID FORM (e.g. ELDERCRAFT^{®} 3.2% LIQUID EUROPEAN BLACK ELDERBERRY EXTRACT)

The product is an elderberry extract, in particular a liquid black elderberry extract, standardized to anthocyanins using ultrafiltration, e.g. to an anthocyanin concentration of 3.2 wt%. This organic solvent free process enriches the active compounds including both the polyphenols and the high molecular weight fractions.
- COLOUR:: Intensive dark red
- APPEARANCE:: Uniform liquid
- TASTE:: Weak for black elderberry
- BOTANICAL NAME:: *Sambucus nigra* L.
- USED PARTS:: Fruits
- INGREDIENTS:: Black elderberry
- DER:: 16 : 1

The term "DER" relates to a drug extraction ratio; when the DER is 16:1, 16 kg elderberry raw material are used to obtain 1 kg ultrafiltrated elderberry extract.

**CHEMICAL VALUES**

| | | |
|---|---|---|
| Soluble solids refract (20°C) Brix | 30.0 - 34.0 | |
| pH - value | 2.90 - 3.50 | |
| Acidity; expressed as citric acid (pH 8.1); (anhydr., IFU 3) | g / kg 30.0 - 50.0 | |
| Density (20° C) | g / ml 1.0500 - 1.1100 | |
| Anthocyanins; expressed as Cya-3-glu (spectrom. by pH-Diff.) | g / kg 32.0 - 42.0 | |
| Polyphenols; expressed as Catechin (Folin Ciocalteaus) | g / kg 42.0 - 81.0 | |

### NUTRITIVE VALUES

Mean values per 100 g extract by analysis or calculation

| | | | |
|---|---|---|---|
| Energy | kcal | 86 | |
| | | kJ | 365 |
| Fat | | g | 0.1 |
| | of which saturates | g | < 0.1 |
| Carbohydrate | | g | 19.2 |
| | of which sugars | g | 6.8 |
| Protein | | g | 1.7 |
| Salt | | g | 0.015 |

### RESIDUES OF PESTICIDES

The processed fruit is conform to the pesticide standards defined in the EC Regulations n. 396/2005 and following amendments.

**RESIDUES OF HEAVY METALS**

| | | |
|---|---|---|
| Arsenic (As) | ppm | < 0.3 |
| Lead (Pb) | ppm | < 0.6 |
| Mercury (Hg) | ppm | < 0.05 |
| Cadmium (Cd) | ppm | < 0.1 |

The product is stable and can be stored for 8 months, preferably stored at a temperature in the range of from -20°C to 7°C, e.g. 0°C to 4°C.

### Example 2: Preparation of ultrafiltrated elderberry extract in a solid form.

The method of preparing an ultrafiltrated elderberry extract in a solid form comprises the method as described in example 1**.** The thus obtained elderberry extract, particularly liquid elderberry extract, is further processed to obtain a solid elderberry extract (Fig. 7B). Optionally, a carrier, such as maltodextrin or gum arabic, is added to the liquid elderberry extract. The obtained liquid extract is dried using a drying method, for example, spray drying and/or lyophilization, preferably spray drying. Optionally, the extract is sieved, e.g. with a sieve having a cut-off at 0.5 mm. The dried extract, e.g. solid extract, is then stored. The method provides a stable extract having a high quality. Furthermore, the method provides an elderberry extract with enriched anthocyanins.

### ULTRAFILTRATED ELDERBERRY EXTRACT IN A SOLID FORM (e.g. ELDERCRAFT^{®} 14% EUROPEAN BLACK ELDERBERRY EXTRACT)

The product is an elderberry extract, in particular a solid black elderberry extract, e.g. a spray dried elderberry extract, standardized to anthocyanins using ultrafiltration, e.g. standardized to an anthocyanin concentration of 14 wt%. This organic solvent free process enriches the active compounds including both the polyphenols and the high molecular weight fractions.
- COLOUR:: Intensive dark red
- APPEARANCE:: Powder, uniform particles, hygroscopic
- TASTE:: Distinctive taste of black elderberry, astringent
- BOTANICAL NAME:: *Sambucus nigra* L.
- USED PARTS:: Fruits
- INGREDIENTS:: Black elderberry, maltodextrin Maltodextrin content: < 5%
- SOLUBILITY:: Soluble in water and water-ethanol mix
- DER:: 64 : 1

The term "DER" relates to a drug extraction ratio; when the DER is 64:1, 64 kg elderberry raw material are used to obtain 1 kg ultrafiltrated elderberry extract.

**CHEMICAL VALUES**

| | | |
|---|---|---|
| Acidity; expressed as citric acid (pH 8.1); (anhydr., IFU 3) | g / kg | 90.0 - 190.0 |
| Moisture; (Loss on Drying at 105° C) | % | 2.0 - 8.0 |
| Particle size; (< 250 µm) (> 60 mesh) | % | 98.0 - 100.0 |
| Anthocyanins; expressed as Cya-3-glu (spectrom. by pH-Diff.) | g / kg | 140.0- 180.0 |
| Polyphenols; expressed as Catechin (Folin Ciocalteaus) | g / kg | 180.0- 350.0 |

**NUTRITIVE VALUES**

| | | | |
|---|---|---|---|
| Mean values per 100 g product by analysis or calculation | | | |
| Energy | | kcal | 343 |
| | | kJ | 1453 |
| Fat | | g | 0.2 |
| | of which saturates | g | < 0.2 |
| Carbohydrate | | g | 76.4 |
| | of which sugars | g | 23.8 |
| Protein | | g | 6.6 |
| Salt | | g | 0.050 |

### RESIDUES OF PESTICIDES

The processed fruit is conform to the pesticide standards defined in the EC Regulation No. 396/2005 and subsequent amendments of the EC Regulation.

### RESIDUES OF HEAVY METALS

Conform to European Regulation 629/2008

| | | |
|---|---|---|
| Lead (Pb) | ppm | < 3 |
| Mercury (Hg) | ppm | < 0.1 |
| Cadmium (Cd) | ppm | < 1 |

The product is stable and can be stored for 36 months.

### Example 3: Elderberry extract is effective against SARS-CoV-2.

It was tested in vitro whether ultrafiltrated elderberry extract, particularly liquid ultrafiltrated elderberry extract, inhibits SARS-CoV-2 in Vero-B4 cells. Elderberry juice (EJ) was compared with ultrafiltrated elderberry extract of the invention, particularly an ultrafiltrated elderberry extract comprising 3.2 wt% anthocyanin (EC3.2). 1 µM Remdesivir was used as a positive control. Elderberry juice is prepared by pressing elderberries and subsequently performing a sterile filtration using a 0.22 µm filter.

Vero B4 cells were maintained in Dulbecco's Modified Eagle's Medium (DMEM) containing 10% (v/v) inactivated fetal calf serum (FCS), 1 mM 1-glutamine, 100 U/mL penicillin, and 100 µg/mL streptomycin. The "Wuhan Type" virus SARS-CoV-2PR-1, isolated from a 61 year old patient, was amplified in Vero B4 cells as described in [5].

Viral titers were determined by an endpoint titration assay. Endpoint of virus infection was analyzed via fluorescence microscopy and viral titer was calculated by the method of Reed and Muench [6]. For titer determination of SARS-CoV-2 virus stocks, A549-ACE2/TMPRSS2 were infected with serial dilutions of the virus stock over 72 h. Afterwards cells were fixed (4% PFA), permeabilized (0.5 % Triton/PBS), blocked (1% BSA/PBS-T) and finally stained with a SARS-CoV-2 NP antibody (Biozol). For the generation of new virus stock, virus containing cell culture supernatant was harvested 72 hours post infection and passed through a 0.45 µm pore-size filter. Virus stocks were stored at -80 °C until further usage. For infection experiments, cells were inoculated with SARS-CoV-2PR-1 (multiplicity of infection (MOI): 2×10-2) for 1 h, washed and further treated with Elderberry Juice (EJ) or Elderberry extract (EC3.2). 72 hpi, virus-containing cell culture supernatants were incubated for 10 min at 95°C and finally used for qRT-PCR analysis. For Western Blot analysis, released virions were purified through 20% (w/v) sucrose cushion (20,000×g, 4°C, 90 min) and then were by SDS-PAGE, transferred onto nitrocellulose membranes, blocked with 3% bovine serum albumin, and incubated with antibodies derived from convalescent SARS-CoV-2 patient sera.

The results of the inhibition experiments using elderberry juice (EJ) in Vero B4 cells are shown in Figure 1 A) and B). As shown in Figure 1, elderberry juice is effective in inhibiting A) viral protein release and B) replication of the virus. Particularly, as shown in Figure 1A) at a dilution of 1:200, the elderberry juice significantly decreases viral protein release. At a dilution of 1:100, viral protein release is effectively inhibited, as no viral protein release is detectable. Surprisingly, elderberry juice is an effective inhibitor of SARS-CoV-2.

The results of the inhibition experiments using an exemplary ultrafiltrated elderberry extract of the invention, particularly liquid elderberry extract EC3.2, in Vero B4 cells are shown in Figure 2 A) and B). As shown in Figure 2, elderberry extract is highly effective in inhibiting A) viral protein release and B) replication of the virus. Particularly, as shown in Figure 2A) at a dilution of 1:400, the elderberry extract juice significantly decreases viral protein release. At a dilution of 1:200, viral protein release is effectively inhibited, as no viral protein release is measurable. As shown in Figure 1 and Figure 2, surprisingly, ultrafiltrated elderberry extract is much more effective in inhibiting SARS-CoV-2 infection than elderberry juice. Advantageously, an ultrafiltrated elderberry extract is highly effective in inhibiting SARS-CoV-2 replication. Without wishing to be bound by any theory, the inventors believe that the ultrafiltration method increases the inhibitory activity of the ultrafiltrated elderberry extract compared to elderberry juice by increasing the concentration of the HMWF.

### Cell viability Assay

The decrease in viral protein release by incubation with elderberry extract is not caused by cell toxicity, but by an antiviral effect. Cell viability was tested using a water-soluble tetrazolium salt (WST)-i assay (Roche) and by neutral red assay according to the manufacturer's instructions. Cells were treated for 72 hours with various inhibitors according to the protocols of the infection experiments. As shown in Figure 4, Vero-B4 cells and A549 ACE2/TMPRSS2+ remain stable and are viable when incubated with elderberry juice (EJ) or elderberry extract (EC 3.2). Particularly, the dilution of 1:200, which shows a strong antiviral effect, does not decrease cell viability compared to untreated cells. Elderberries, particularly elderberry juice and elderberry extract, surprisingly have an antiviral effect against SARS-CoV-2. The antiviral effect against SARS-CoV-2 of elderberry extract is even stronger than the antiviral effect of elderberry juice. Accordingly, the elderberry extract of the invention has a surprising antiviral effectivity against SARS-CoV-2 infections.

### Example 4: The elderberry extract has an antiviral activity against SARS-CoV-2 variants of concern Alpha and Beta

The ultrafiltrated elderberry extract was tested for its activity against SARS-CoV-2 wildtype, SARS-CoV-2 Alpha and SARS-CoV-2 Beta using A549-cells expressing ACE2 and TMPRSS2. The virus strains SARS-CoV-2 Alpha and Beta were obtained from Prof. Michael Schindler (University Hospital, Tübingen). Thereby, the Alpha variant (210416_UKv) was isolated from a throat swab collected in April 2021 at the Institute for Medical Virology and Epidemiology of Viral Diseases, University Hospital Tübingen, from a PCR-positive patient. In total, 200 µl of patient material was diluted in medium and used to directly inoculate 150.000 CaCo-2 cells in a six-well plate. 48h post-infection, the supernatant was collected, centrifuged, and stored at -80 °C. After two consecutive passages, the supernatant was tested by qRT-PCR confirming the presence of the N501Y point mutation. Finally, NGS confirmed that the clinical isolate belongs to the lineage B.1.1.7.SARS-CoV-2 Beta was generated as described in [7].

Viral titers were determined by an endpoint titration assay. Endpoint of virus infection was analyzed via fluorescence microscopy and viral titer was calculated by the method of Reed and Muench [6]. For titer determination of SARS-CoV-2 virus stocks, A549-ACE2/TMPRSS2 were infected with serial dilutions of the virus stock over 72 h. Afterwards cells were fixed (4% PFA), permeabilized (0.5 % Triton/PBS), blocked (1% BSA/PBS-T) and finally stained with a SARS-CoV-2 NP antibody (Biozol). For the generation of new virus stock, virus containing cell culture supernatant was harvested 72 hours post infection and passed through a 0.45 µm pore-size filter. Virus stocks were stored at -80°C until further usage. For infection experiments, cells were inoculated with SARS-CoV-2 Alpha or SARS-CoV-2 Beta (multiplicity of infection (MOI): 2×10-2) for 1 h, washed and further treated with Elderberry Extract (EC3.2). 72 hpi, virus-containing cell culture supernatants were incubated for 10 min at 95°C and finally used for qRT-PCR analysis.

### Cell culture

A549-cells expressing ACE2 and TMPRSS2 were generated by retroviral transduction as described in [5] and cultivated in RPMI 1640 medium containing 10% (v/v) inactivated fetal calf serum (FCS), 2 mM l-glutamine, 100 U/mL penicillin, 100 µg/mL streptomycin and 100 µg/mL blastomycin.

### Determination of the amount of viral RNA copies from released viruses by qRT-PCR

Virus was quantified by real-time PCR AgPath-ID One-Step RT-PCR Kit from Ambion (Cat: 4387424) software v2.3 (applied Bioscience). PCR primers were used according to [5].

### Results:

The ultrafiltrated elderberry extract was highly effective in all tested SARS-CoV-2 strains, including SARS-CoV-2 wildtype, SARS-CoV-2 Alpha and SARS-CoV-2 Beta with a comparable efficacy. Ultrafiltrated elderberry extract showed similar inhibitory activity to all 3 variants (SARS-CoV-2 wildtype, SARS-CoV-2 Alpha and SARS-CoV-2 Beta in A549-cells expressing ACE2 and TMPRSS2.

As shown in Figure 3 ultrafiltrated elderberry extract is also effective in inhibition of the viral replication of variants SARS-CoV-2 Alpha (Fig. 3 B) and SARS-CoV-2 Beta (Fig. 3 C) as in SARS-CoV-2 wildtype (Fig. 3 A).

### Example 5: Polysaccharide isolation and characterization.

The aim of this experiment was to isolate and characterize the polysaccharide fraction from a dried ultrafiltrated elderberry extract as described in example 2. A series of ethanol precipitations combined with an ion exchange purification was performed to generate polysaccharide fractions.

### Methods

100 g of dried elderberry extract was dissolved in 2.5 litres of ultra-pure water (UPW) with stirring, then chilled ethanol was added to 75 % (v/v). The solution was stirred vigorously, covered with a cling-film, and left overnight at 5 °C in a cold room.

A deep purple coloured precipitate formed and was collected by successive centrifugations (20 mins at 10 000 × g in a Sorvall cooled ultrafuge at 5 °C). The pellets were resuspended in UPW and samples taken for total sugar and total anthocyanin tests. The supernatants were combined and retained. The pelleted material was then re-precipitated at 75 % (v/v) ethanol using the same procedure. The second precipitate was resuspended in UPW then dialysed overnight against an excess of UPW. The crude polysaccharide sample (CPS) was frozen and then freeze dried.

Samples were assessed for anthocyanin content and polysaccharide content using the phenol sulphuric acid method. The original elderberry extract contained high anthocyanin content (~16.63 g CGE/ 100 g) and sugar content (~3.79 g Glc equivalents/ 100 g). However, the total sugar value includes sugars attached to the anthocyanins. The majority of anthocyanins remained in the first ethanol supernatant and the sequential precipitations enriched the polysaccharide content of the pelleted material by 4.6-fold and 12.1-fold respectively over the original extract.

To further purify the crude polysaccharide sample, 500 mg of the sample was resuspended in 10 mL of 100 mM Tris HCl pH 8.0 and applied to a 100 mL column of DEAE-Sepharose equilibrated in the same buffer. The column was eluted with 2 × 50 ml volumes of Tris buffer to give the unbound 1 and unbound 2 samples then sequentially with 50 mL of 0.25 M NaCl, 0.5 M NaCl then 3 × 50 mL of 1 M NaCl in Tris buffer. The polysaccharide (as noted as total sugar content) eluted in two main peaks, the unbound fraction 1 and the 0.25 M NaCl bound fraction (see Fig. 5). The anthocyanins were mainly found in the 0.5 M and 1 M NaCl fractions. The procedure was repeated 8 times and the combined unbound 1 and 0.25 M NaCl bound fractions were precipitated with 75 % (v/v) ethanol, resuspended in UPW and freeze dried.

The CPS, unbound fraction 1 and 0.25 M bound fractions were acid hydrolysed and their monosaccharide composition determined using HPAEC.

### Results

3 fractions of polysaccharides from ultrafiltrated dry black elderberry were generated as shown in Table 1.

**Table 1: Polysaccharide fractions of ultrafiltrated elderberry extract.**

| **Name** | **Description** |
|---|---|
| EC15 | Ultrafiltrated dried Black Elderberry Extract 15% |
| CPS | Crude Polysaccharide fraction, obtained from the ethanol precipitation step |
| UNBOUND | Flow-through polysaccharide rich fraction obtained from ion exchange purification step (UB1 and UB2) |
| BOUND | Bound polysaccharide rich fraction from to the ion exchange purification step (0.25M) |

The monosaccharide analysis revealed that the BOUND fraction was enriched in galacturonic acid and contained galactose, arabinose and rhamnose (see Fig. 6), which is similar to that reported previously for pectins extracted from elderberry. The bound sample had notably reduced amounts of glucose, galactose, xylose and mannose compared to the CPS, which indicated the removal of neutral polysaccharides by the ion exchange procedure. The higher glucose and xylose content of the CPS may also reflect the presence of these sugars in the anthocyanins [4].

### Example 6: The ultrafiltrated elderberry extract and its fractions has an immunostimulatory effect, stronger than state of the art ethanolic extracts.

The samples generated in example 5 (CPS, BOUND and UNBOUND) together with the ultrafiltrated spray-dried elderberry extract (EC15) generated as described in example 2 where further analysed for their immunostimulatory activity on human immune cells. Additionally, the immunostimulatory activity of a conventional ethanolic elderberry extract (EE25) as described in example 8 containing 25% anthocyanin and no polysaccharides (in the form of dietary fibers) was evaluated.

Ultrafiltrated spray-dried elderberry extract, standardized to 15 % anthocyanins (EC15), CPS, BOUND, UNBOUND and EE25 were generated as described in example 5. The powders were reconstituted in 1x PBS, steril filtered using a Millex-GP 0.22 µm PES Membrane (Merck Millipore Ltd. Tullagreen, IRL) and stored at +4 °C.

### Generation of human monocyte-derived dendritic cells:

Human monocyte-derived immature DCs (iDCs) were generated from leukoreduction system chambers (LRSCs) as previously described [8]. Briefly, peripheral blood mononuclear cells (PBMCs) from healthy donors were isolated by density gradient centrifugation using Lymphoprep (Axis-Shield/Alere Technologies AS, Oslo, Norway), followed by plastic adherence on tissue culture dishes (Falcon, Fisher Scientific, Germany).

The non-adherent fraction (NAF) containing T cells was removed and stored at -80 °C in 11 % human serum albumin (HSA; CSL Behring, King of Prussia, Pennsylvania, USA), 10 % glucose (Merck, Darmstadt, Germany) and 20 % dimethyl sulfoxide (DMSO; Sigma-Aldrich, St. Louis, Missouri, USA), for subsequent co-culture experiments.

The adherent fraction was cultured for 6 days in DC medium consisting of RPMI 1640 (Lonza, Basel, Switzerland), supplemented with 1 % (v/v) heat-inactivated human serum type AB (Sigma-Aldrich), 1 % (v/v) penicillin streptomycin L-glutamine (Sigma-Aldrich) and 10 mM HEPES (Lonza), in the presence of 800 IU/ml (day 0) or 400 IU/ml (day 3 and day 5) recombinant human GM-CSF and 250 IU/ml (day 0,3 and 5) recombinant human IL 4 (both from Miltenyi Biotec, Bergisch Gladbach, Germany).

On day 6, cells were treated with 10 µg/ml of spray dried ultrafiltrated dry Elderberry extract (EC15), CPS, BOUND,UNBOUND, and EE25, respectively for 24 h. As maturation control iDCs were treated with 100 ng/ml LPS (Sigma Aldrich).

### Mixed lymphocytes reaction (MLR):

On day 7, the above described and pretreated human iDCs were used for phenotypical and functional analyses. On day 7, pre-treated iDCs were co-cultured at different ratios with 4^{∗}105 allogeneic NAF cells, and ultrafiltrated elderberry extract 15 % (EC15), CPS, BOUND, UNBOUND, and EE25 were added freshly, at the same concentration as used on day 6. As control, untreated iDC were also co-cultured at different ratios with 4^{∗}105 allogeneic NAF cells, without the addition of Elderberry derived substances. Subsequently, co-cultures were incubated in 96-well cell culture plates (Falcon, Fisher Scientific) for 72 h, in a total volume of 200 µl/well and 10 µg/ml of Elderberry derived substances. After 3 days, cell-free supernatants were collected from each condition and analyzed for their cytokine secretion levels, as previously described [8]. To analyze the T cell stimulatory capacity of DCs treated with different Elderberry derived substances, cells were pulsed with 3H-thymidine (1 µC/well; PerkinElmer, Rodgau, Germany) for 16 - 20 h, to determine allogeneic T cell proliferation, a Thymidine incorporation has been assessed using a Wallac 1420 Victor2 Microplate Reader (PerkinElmer). As a proliferation control, NAF cells, containing T cells, were incubated together with DynabeadsTM Human T-Activator CD3/CD28 (Thermo Fisher Scientific, Waltham, Massachusetts, USA).

### Cytometric bead array (CBA):

The secretion of cytokines into the supernatant of co-cultures, was determined using the cytometric beads array (CBA) LEGENDplex^{™} HU Th Cytokine Panel (BioLegend, San Diego, California, USA), according to the manufacturer protocol.

### Flow cytometric analyses:

For the phenotypical characterization of human DCs, the following monoclonal antibodies were used (all purchased from BioLegend, unless stated otherwise): CD11c-APC (clone 3.9), CD14-BV510 (clone M5E2), CD40-PECy7 (clone 5C3), CD80-BV421 (clone 2D10) CD83-PE (clone HB15e), CD86-FITC (clone FUN-1; BD) and MHCII-APCCy (clone L243). For dead cell discrimination, 7-AAD viability staining solution (ThermoFisherScientific) was added directly to the surface staining. The surface staining was performed for 30 min at 4°C in the dark in DPBS.

### Statistics:

All statistical analyses were performed with GraphPad Prism 8.3, using the one way ANOVA (Figures 8A, 8B, 8C), Figures 10, 11A - 11F) and the two way ANOVA (Figure 9A -9E). Data are presented as mean values including the SEM. ^{∗}p < 0.05; ^{∗∗}p < 0.01; ^{∗∗∗}p < 0.001; and ^{∗∗∗∗}p < 0.0001 were considered statistically significant.

### Results

In order to evaluate the potential immuno-stimulatory capacity of Elderberry derived substances, Dendritic cells (DCs), which are the most potent antigen presenting cells (APCs) and the only APCs able to induce also naive T cells, have been incubated with different Elderberry derived substances. The phenotypical changes due to the treatment were analyzed by flow cytometry. Subsequently, their immuno-stimulatory properties have been analyzed using Mixed Lymphocyte Reaction (MLR) analyses, investigating (i) the cytokine secretion and (ii) the T cell stimulatory capacity mediated by these DCs.

Results regarding cytokine secretion levels are depicted in Figures 8A, 8B, 8C. Immature DCs (iDCs), which per se are poor T cells stimulators, were incubated with different Elderberry derived substances, i.e. with EC15, CPS, BOUND, UNBOUND, or EE25, and the cytokine secretion into the MLR supernatant was analyzed.

As shown in Figure 8A, EC15 induced an increased IL-6 release into the supernatant, when compared with untreated iDCs. The cytokine secretion was even further induced when cells were incubated with the CPS, BOUND or UNBOUND fractions. Thus, these data clearly indicate that EC15 and fractions CPS, BOUND or UNBOUND induced highly elevated cytokine expression levels, when compared to iDCs. No increased IL-6 levels were detected with EE25 treatment, when compared to iDCs. Without wishing to be bound by any theory, it appears that the polysaccharides present in fraction CPS, BOUND and UNBOUND play an important role in the observed effects such as the immunomodulatory effect and antiviral effect.

Similar results were obtained, when TNF-α secretion was analyzed (Figure 8B). In this case EC15 induced an increase in TNF-a secretion, in comparison to untreated control (i)DCs. Fractions CPS, BOUND and UNBOUND, induced a highly increased cytokine secretion, in comparison to iDCs. No significant TNF-α secretion was induced with EE25 treatment, when compared to iDCs.

Figure 8C shows data regarding IFN-γ secretion levels. The secretion of IFN-y in the EC15 and EE25 treatment condition is not significantly induced, when compared to iDCs. In sharp contrast, the CPS, BOUND and UNBOUND fractions increased IFN-y secretion levels of in a highly significant way.

Thus, in summary these results clearly show that the elderberry extract and the derived fractions CPS, BOUND and UNBOUND, increase the secretion of specific pro-inflammatory cytokine levels and therefore induce potent immune responses. In contrast, the treatment with EE25 has no effect on cytokine expression levels, when compared to iDCs and, thus, does not induce a potent immune response.

In order to analyze the T cell stimulatory capacity of DCs, treated with different Elderberry derived substances, the MLR co-culture assay has been used. Thus, cells were pulsed with 3H-thymidine for 16 - 20 h, to determine allogeneic T cell proliferation. As shown in Figures 9A - 9E, iDCs induced only a week T cell proliferative response. In contrast, EC15 (Figure 9A) and the fractions CPS (Figure 9B, BOUND (Figure 9C), and UNBOUND (Figure 9D) induced a strong and statistically highly significant T cell specific immune response, when compared to iDCs. Without wishing to be bound by any theory, it appears that the polysaccharides enriched in fraction CPS, BOUND and UNBOUND provide an immunomodulatory effect and antiviral effect. As shown in Figure 9E, EE25 did not induce a significant T cell response, when compared to iDCs.

The phenotypic impact of EC15, CPS, BOUND, UNBOUND and EE25 on immature DCs regarding the surface expression of specific surface molecules, was also assessed (11A- 11F). The treatment with the different extracts had no effect on cell viability (Figure 10). CD11c expression was increased in EC15, CPS, BOUND, UNBOUND treated cells, while this molecule was not overexpressed in untreated iDC or EE25 treated iDCs, indicating that EE25 does not induce DC maturation (Figure 11A). The expression of maturation markers, including HLA-DR, CD80 and CD86 are highly significantly upregulated by the treatment of iDCs with EC15, CPS, BOUND and UNBOUND (Figure 11B, 11C and 11D). Also, the costimulatory molecule CD40 and the important mDC-specific CD83 molecule are highly upregulated (Figure 11E and 11F), lower row). In sharp contrast, EE25 treatment of iDC had no effect on the expression of typical maturation markers and costimulatory molecules. In summary, the elderberry fractions EC15, CPS, BOUND and UNBOUND induce the phenotypic maturation of DCs which correlates with the improved T cell stimulatory capacity and the increased cytokine levels. In sharp contrast, EE25 does not induce these phenotypical and functional properties.

Conclusively, the polysaccharides present in the extract of the invention are an important factor for the immunostimulatory effects of elderberry extracts. Conventional extracts do not contain high amounts of polysaccharides (as shown in example 8) and are less active.

### Example 7: Qualitative and quantitative determination of anthocyanins, e.g. Cyanidin 3-Glucoside, by HPLC.

The assay was used to determine total anthocyanin contents in an ultrafiltrated liquid elderberry extract standardized to 3.2% anthocyanins as described in example 1. It is a reversed-phase high performance liquid chromatography (RP-HPLC) method that was used to calculate the total anthocyanin content of an extract as well as to examine the qualitative "fingerprint" (chromatogram) of the anthocyanins present in the extract.

Anthocyanins were separated by RP-HPLC and monitored at 520 nm to obtain individual chromatograms of fruits or products. A standard curve was built with cyanidin-chloride at 525 nm. The area under the curve of the sample was related to the standard curve to determine the total anthocyanin concentration. The HPLC-system used comprised a computer with HPLC software, a UV-VIS detector, a RP-column C18, an autosampler, gradient pumps, thermostat; filtration apparatus with filters; volumetric flasks; volumetric pipettes; analytical balance. The reagents used were cyanidin-chloride; water, HPLC grade; acetonitrile, HPLC grade; triethylamine (TEA); perchloric acid (70%); ethanol (95%); methanol (95%); hydrochloric acid (32%); and phosphoric acid (75%).

### HPLC Conditions

The HPLC conditions are similar to other published methods e. g. IFU No. 71 as established by International Fruit and Vegetable Juice Association.

| | |
|---|---|
| Flow Rate | 0.8 mL/min |
| Column | Temperature 42° C |
| Injection Volume | 30 µL |
| Detection wavelength | 520 nm sample, 525 nm standard |
| Analysis Time | 47 min |
| Gradient | Elution profile: |
| | 0 - 3 min 8% to 12% B (linear); |
| | 3 - 15 min 12% - 18% B (linear); |
| | 15 - 29 min 18% to 33% B (linear); |
| | 29 - 30 min 33% to 100% B; |
| | 30 - 37 min 100% B; |
| | 37 - 38 min 100% B. |

### Preparation and procedure

Injection solution: 3.13 g perchloric acid were mixed with 500 mL water, 100 mL ethanol were added in a 1000 mL volumetric flask and filled up with water to 1000 mL.

Solvent A: 4.42 g perchloric acid were mixed with 500 mL water, 2 mL TEA are added and filled up to 1000 mL with water. pH was adjusted at 1.5 with phosphoric acid.

Solvent B: 400 mL of solvent A (before adjusting to pH 1.5 with phosphoric acid) were mixed with 600 mL acetonitrile.

Standard preparation: 5 standards (from 20 to 200 mg/L) were prepared from a fresh solution cyanidin-chloride with 200 mg/L (=1 mg cyanidin-chloride in 5 mL methanol with 1% HCl) by diluting with appropriate quantities of injection solution. Standard solution was filtered and 30 µl were injected. The evaluation of the calibration curve was made at the absorbance maximum of cyanidin-chloride (525 nm).

Sample preparation: The extract was diluted with 140 µl injection solution and filtered. The injection volume was 30 µl, absorbance was monitored at 520 nm.

### Calculation

All data was evaluated using the cyanidin-chloride calibration curve. A conversion factor of 1.393 was used to convert the result from cyanidin-chloride into cyanidin-3-glucoside.

### Results

The chromatogram of the analysis is shown in figure 12. Three independent peaks can be identified corresponding to the anthocyanins cyanidin-3-sambubioside-5-glucoside (peak 1), cyanidin-3-sambubioside (peak 2) and cyanidin-3-glucoside (peak 3). A total content of content of 34.1 g/kg anthocyanin expressed as cyanidin 3-glucoside was determined. **Example 8:** Comparison of an ultrafiltrated elderberry extract of the invention with a conventional elderberry ethanol extract.

A dried powder of ultrafiltrated elderberry extract standardized to at least 14% anthocyanins was compared to a conventional commercially available ethanol extract standardized to 25% anthocyanins produced using an ethanol extraction. Ethanolic extraction is the common method of preparation of elderberry extracts. Water-ethanol mixtures are used to increase anthocyanin yields during extraction and anthocyanin are then purified using resins (columns). The here described analysis determined the levels of nutrients, micronutrients, vitamins, fiber and minerals of both preparations.

### Methods

The analysis was performed by external accredited laboratories using validated methods shown in table 2.

**Table 2: methods used to compared to composition of the extract of the invention with an ethanol extract.**

| **Anaysis** | **Method** |
|---|---|
| Fiber | Chelab Methode MP 2135 rev 4 (AOAC 991.43 1994) |
| Potassium | ICP-OES |
| Natrium | ICO-OES |
| Calcium | ICP-OES |
| Magnesium | ICP-OES |
| Phosphor | ICP-OES |
| Copper | ICP-MS |
| Iron | ICP-OES |
| Manganese | ICP-MS |
| Zinc | ICP-MS |
| Protein | N × 6,25 |
| Ash | |
| Vit B1 | LC-MS/MS |
| Vit B2 | LC-MS/MS |
| Vit PP | LC-MS/MS |
| Vit B6 | LC-MS/MS |
| Vit B12 | LC-MS/MS |
| Vitamin C | HPLC-DAD |

### Results

The comparison of the ultrafiltrated elderberry extract and the ethanol extract showed that the ultrafiltration process is retaining bioactive components better than extraction processes using ethanol. A summary of the results is shown in Figure 13.

Dietary fibers of elderberry, which are immunostimulatory are not detectable in the extract produced with ethanol extraction but are present up to 7.5% in the elderberry extract produced using ultrafiltration. Vitamin B2, Vitamin PP, Vitamin C, and Vitamin B6 are present at higher concentration in the elderberry extract produced using ultrafiltration than the products produced using an ethanolic extraction process.

Copper, iron, manganese and zinc are present at higher concentration in the elderberry extract produced using ultrafiltration than the products produced using an ethanolic extraction process.

**Table 3: Comparison of the ultrafiltrated elderberry extract of the invention with an ethanol extract.**

| | Ultrafiltrated elderberry extract (mg/kg) | Ethanol Extraction (mg/kg) |
|---|---|---|
| Potassium (K) | 13500 | 1290 |
| Natrium (Na) | 130 | 190 |
| Calcium (Ca) | 1200 | 101 |
| Magnesium (Mg) | 1750 | 99 |
| Phosphor (P) | 1390 | 430 |
| Copper (Cu) | 11.6 | 1.3 |
| Iron (Fe) | 95.2 | 33.7 |
| Manganese (Mn) | 15.3 | 1.7 |
| Zinc (Zn) | 8.3 | 1.4 |
| Protein | 6103 | 4891 |
| Fiber | 7483.6 | < 0.5 |
| Ash | 2817 | 275 |
| Vit. B1 (Thiamin) | 3.0 | 0.5 |
| Vit. B2 (Riboflavin) | 8.7 | 7.3 |
| Vit. PP (Niacin) | 95.8 | 33.1 |
| Vit. B12 | 0.00329 | 0.00070 |
| Vit. C | 26.2 | 19.2 |
| Vit. B6 (Pyridoxin) | 9.9 | 8.9 |

Figure 13 shows a comparison of the ultrafiltrated elderberry extract of the invention and an ethanol extract. A solid ultrafiltrated elderberry extract having an anthocyanin concentration of 14 wt% was compared to an ethanol extract. The ethanol extract has an anthocyanin concentration of 25 wt%. For easier comparison the results of the analysis were calculated to an anthocyanin concentration of 14 wt%.

### REFERENCES

[1] Kinoshita E, Hayashi K, Katayama H, Hayashi T, Obata A. Anti-influenza virus effects of elderberry juice and its fractions. Biosci Biotechnol Biochem. 2012;76(9):1633-8.
[2] Tiralongo E, Wee S, Lea R. Elderberry supplementation reduces cold duration and symptoms in air travelers: a randomized, double-blind placebo-controlled clinical trial. Nutrients. 2016;8,182.
[3] Yang B, Lin X, Yang C, Tan J, Li W, Kuang H. Sambucus Williamsii Hance Promotes MC3T3-E1 Cells Proliferation and Differentiation via BMP-2/Smad/p38/JNK/Runx2 Signaling Pathway. Phytother Res. 2015 Nov;29(11):1692-9.
[4] da Silva RFR, Barreira JCM, Heleno SA, Barros L, Calhelha RC, Ferreira ICFR. (2019) Anthocyanin profile of elderberry juice: A natural-based bioactive colouring ingredient with potential food application. Molecules. 24(13):2359.
[5] Große M, Ruetalo N, Layer M, Hu D, Businger R, Rheber S, Setz C, Rauch P, Auth J, Fröba M, Brysch E, Schindler M, Schubert U (2021) Quinine Inhibits Infection of Human Cell Lines with SARS-CoV-2; Viruses 2021, 13(4), 647; https://doi.org/10.3390/v13040647.
[6] REED, L. J. & MUENCH, H. A SIMPLE METHOD OF ESTIMATING FIFTY PER CENT ENDPOINTS12. American Journal of Epidemiology 27, 493-497, doi:10.1093/oxfordjournals.aje.a118408 (1938)).
[7] Becker, M.; Dulovic, A.; Junker, D.; Ruetalo, N.; Kaiser, P. D.; Pinilla, Y. T.; Heinzel, C.; Haering, J.; Traenkle, B.; Wagner, T. R.; Layer, M.; Mehrlaender, M.; Mirakaj, V.; Held, J.; Planatscher, H.; Schenke-Layland, K.; Krause, G.; Strengert, M.; Bakchoul, T.; Althaus, K.; Fendel, R.; Kreidenweiss, A.; Koeppen, M.; Rothbauer, U.; Schindler, M.; Schneiderhan-Marra, N., Immune response to SARS-CoV-2 variants of concern in vaccinated individuals. Nat Commun 2021, 12, (1), 3109.
[8] Pfeiffer I A, Zinser E, Strasser E, Stein M F, Dörrie J, Schaft N, Steinkasserer A, Knippertz I., Leukoreduction system chambers are an efficient, valid, and economic source of functional monocyte-derived dendritic cells and lymphocytes Immunobiology, 2013 218(11) 1392-1401.

The features of the present invention disclosed in the specification, the claims, and/or in the accompanying figures may, both separately and in any combination thereof, be material for realizing the invention in various forms thereof.

## Claims

1. An elderberry extract for use in a method of preventing or treating a SARS-CoV-2 infection, wherein said elderberry extract is an ultrafiltrated elderberry extract, preferably an ultrafiltrated black elderberry extract, optionally of *Sambucus nigra* L and/or *Sambucus canadensis* L.

2. The elderberry extract for use according to claim 1, wherein said extract comprises one or more anthocyanins, preferably selected from the group consisting of cyanidin 3-glucoside, cyanidin 3-sambucoside, cyanidin 3-sambubioside 5-glucoside, and combinations thereof, and/or
comprises one or more polyphenols, preferably selected from rutin, quercetin, catechin, and epicatechin.

3. The elderberry extract for use according to claim 1 or 2, wherein said extract has an anthocyanin concentration of at least 1 wt%, preferably at least 3 wt%; wherein, optionally, said extract has an anthocyanin concentration in the range of from 2.5 wt% to 20 wt%, preferably from 3 wt% to 18 wt%, e.g. 3.2 wt%, 4 wt%, 7 wt%, 10 wt%, 14 wt%, or 15 wt%.

4. The elderberry extract for use according any one of the foregoing claims, wherein said extract has a high molecular weight fraction concentration of at least 1 wt%, preferably at least 3 wt%, more preferably in the range of from 5 wt% to 10 wt%, wherein, preferably, said high molecular weight fraction has a molecular weight in a range of from 5 kDa to 2 MDa,
wherein, optionally, said high molecular weight fraction comprises protein(s), dietary fibers, and/or polysaccharide(s).

5. The elderberry extract for use according any one of the foregoing claims, wherein said extract has a dietary fiber concentration of at least 1 wt%, preferably of at least 2 wt%, wherein, optionally, said dietary fiber is selected from pectins and polysaccharides, e.g. acidic polysaccharides, and combinations thereof.

6. The elderberry extract for use according any one of the foregoing claims, wherein said extract is in a liquid form or in a solid form,
wherein, optionally, said extract in a solid form has an anthocyanin concentration of at least 1.5 wt%, preferably of at least 10 wt%.

7. The elderberry extract for use according any one of the foregoing claims, wherein said extract is an extract ultrafiltrated with an ultrafiltration membrane having a molecular weight cut-off of at least 400 Da, preferably of at least 500 Da, more preferably of at least 1000 Da.

8. The elderberry extract for use according to any one of the foregoing claims, wherein, in said use, said extract is administered to a patient in need thereof in the form of a solid extract at a dose in the range of from 30 mg to 10.000 mg per day, preferably at least 250 mg per day, more preferably at least 900 mg per day; or in the form of a liquid extract at a dose in the range of from 100 µL to 1 L per day, preferably 1 mL to 100 mL per day, more preferably at least 5 mL per day.

9. The elderberry extract for use according to any one of the foregoing claims, wherein, in said use, said extract is administered to a patient in need thereof in the form of a formulation selected from a capsule; a tablet, e.g. an effervescent tablet or a chewable tablet; a syrup; a sachet; gummies; a lozenge; a shot; a gel; a confection; a spray, e.g. an oral spray; a powder; granules; a solution; a suspension; a dispersion; an emulsion; a bar; a drink; a liquid concentrate; an aerosol; or drops;
wherein, optionally, said formulation further comprises, in addition to said extract, an excipient, e.g. a pharmaceutically acceptable excipient, a thickening agent, a sweetener, a sugar, a starch, a preservative, a flavoring agent, a nutritional oil such as a vegetable oil or fish oil, water, a milk such as a cow's milk, a soya milk, or an almond milk, a black elderberry juice or other fruit juice, a vegetable juice, and/or a colorant.

10. The elderberry extract for use according to any one of the foregoing claims, wherein said extract stimulates increased expression of IL-6, TNF-a, and/or IL-23, preferably IL-6, TNF-a, and IL-23, in immune cells, and/or
wherein said extract inhibits a viral replication of said SARS-CoV-2.

11. The elderberry extract for use according any one of the foregoing claims, wherein, in said use, said extract is administered to a patient in need thereof at one or several stages selected from
- a healthy stage;
- an asymptomatic disease stage;
- a mild or moderate disease stage;
- a severe disease stage; and
- a critical disease stage.

12. The elderberry extract for use according any one of the foregoing claims, wherein said SARS-CoV-2 infection is an infection with a SARS-CoV-2 wildtype, preferably severe acute respiratory syndrome coronavirus 2 isolate Wuhan-Hu-1, and/or a SARS-CoV-2 variant, preferably alpha variant or beta variant.

13. The elderberry extract for use according any one of the foregoing claims, wherein said SARS-CoV-2 variant is selected from alpha variant, beta variant, gamma variant, and delta variant, and/or is selected from
B.1.1.7 variant, B.1.351 variant, P.1 variant, B.1.617 variant, B.1.617.1 variant, B.1.617.2 variant, B.1.617.3 variant, B.1.427 variant, B.1.429 variant, B.1.525 variant, B.1.526 variant, P.2 variant, and any combination thereof;
wherein, preferably, said SARS-CoV-2 variant is selected from alpha variant and beta variant.

14. A composition, preferably pharmaceutical composition, comprising an elderberry extract, wherein said elderberry extract is an ultrafiltrated elderberry extract, optionally an elderberry extract as defined in any one of claims 1-13;
wherein, optionally, said extract has an anthocyanin concentration of at least 1 wt%, preferably of at least 3 wt%, e.g. in the range of from 2.5 wt% to 20 wt%, preferably from 3 wt% to 18 wt%;
wherein, optionally, said extract has a high molecular weight fraction concentration of at least 1 wt%, preferably of at least 3 wt%, wherein, preferably, said high molecular weight fraction has a molecular weight in a range of from 5 kDa to 2 MDa;
wherein, optionally, said extract comprises dietary fiber(s) in a concentration of at least 1 wt%, preferably of at least 2 wt%;
wherein, optionally, said composition further comprises an excipient, e.g. a pharmaceutically acceptable excipient, a sweetener, a sugar, a starch, a preservative, a flavoring agent, a nutritional oil such as a vegetable oil or fish oil, water, a milk such as a cow's milk, a soya milk, or an almond milk, an elderberry juice or other fruit juice, a vegetable juice, and/or a colorant, preferably a pharmaceutically acceptable excipient.

15. A method of preparing an ultrafiltrated elderberry extract, comprising the steps:
i) providing one or more elderberries; wherein, preferably, said one or more elderberries comprise black elderberries; wherein, more preferably, said one or more elderberries comprise *Sambucus nigra L* and/or *Sambucus canadensis L;*
ii) performing an ultrafiltration using an ultrafiltration membrane having a molecular weight cut-off of at least 400 Da, preferably of at least 500 Da, more preferably of at least 1000 Da;
iii) obtaining an ultrafiltrated elderberry extract.
